# EUROPEAN PATENT SPECIFICATION

(11) **EP 3 357 415 B1**
(45) Date of publication and mention of the grant of the patent: **07.10.2020**
(21) Application number: 18154954.4
(22) Date of filing: 02.02.2018
(51) Int. Cl.: A61B 5/00

(54) **ELECTRONIC DEVICE CAPABLE OF MEASURING BIOMETRIC INFORMATION**
ELEKTRONISCHE VORRICHTUNG ZUR MESSUNG BIOMETRISCHER INFORMATIONEN
DISPOSITIF ÉLECTRONIQUE POUVANT MESURER DES INFORMATIONS BIOMÉTRIQUES

(30) Priority: 03.02.2017 KR 20170015700
(43) Date of publication of application: 08.08.2018
(73) Proprietor: Samsung Electronics Co., Ltd., Gyeonggi-do 16677 (KR)
(72) Inventor: JEONG, Injo, Yongin-si (KR); KIM, Younghwan, Hwaseong-si (KR); BYEON, Hyungsup, Suwon-si (KR); LEE, Seung-Eun, Seoul (KR); CHUN, Jae-Bong, Suwon-si (KR); HA, Jungsu, Osan-si (KR); CHOI, Bokun, Seoul (KR)
(74) Representative: Nederlandsch Octrooibureau

(56) References cited:
- KR-U- 20160 002 493
- US-A1- 2012 022 385
- US-A1- 2014 081 118
- US-A1- 2017 000 415

## Description

### BACKGROUND

### 1. Field of the Disclosure

The present disclosure relates generally to an electronic device capable of measuring biometric information.

### 2. Description of the Related Art

An electronic device capable of measuring biometric information may require at least one electrode having a high electrical conductivity. For example, a metal material may be utilized as a material of the electrode having a high electrical conductivity.

However, metal has a disadvantage that it may not be applied to a display region of a display because it is an opaque material, even though its electrical conductivity is excellent. The area of the display may be reduced when an electrode made of a metal material is applied to the display region.

Alternatively, an electronic device capable of measuring biometric information may utilize a transparent electrode.

However, when a transparent electrode is used as an electrode of an electronic device, it is necessary to provide a structure in which the transparent electrode is electrically connected to a printed circuit board (e.g., a control circuit) using a flexible printed circuit board (FPCB), and the electronic device, which is applied with the transparent electrode by such a structure, may require a waterproof structure.

Due to the waterproof structure of the electronic device, the bezel region of the electronic device may be enlarged, or limitations may be imposed on the design appearance of the electronic device.

US 2017/000415 A1 discloses a device to be mounted on a wrist of a wearer. The device also includes a first electrical contact disposed on an inner surface of the mount and configured to contact skin at a first external body surface. The device also includes a second electrical contact disposed on an outer surface of the mount and configured to be contacted by skin of a second external body surface.

KR 2016 0002493 U discloses a wearable healthcare device including a lower electrode mounted on the bottom of the housing and contacting the skin of the wrist, and, mounted on a side or upper surface of the housing, an upper electrode contacting the finger of the opposite hand.

US 2012/022385 A1 discloses an electrocardiographic signal detection device including at least one pair of electrodes that detect electrical signals of a living body; an insulating film disposed on the at least one pair of electrodes, the insulating film having a living body contact surface; a differential amplifier that generates an electrocardiographic signal.

### SUMMARY

The present disclosure has been made to address at least the above-mentioned disadvantages and to provide at least the advantages described below.

The invention is defined by the subject-matter of the independent claim 1.

Particular embodiments of the invention are set out in the dependent claims. Aspects of the present disclosure, examples and technical descriptions of apparatuses, products and/or methods in the description and/or drawings which are not covered by the claims are presented not as embodiments of the invention but as background art or examples useful for understanding the invention.

Accordingly, an aspect of the present disclosure provides an electronic device that enables a metal electrode to be used instead of an FPCB in electrical connection between conductive regions, so that a waterproof structure may become unnecessary.

According to an aspect of the present disclosure, an electronic device is provided that enables a transparent electrode to be a first electrode, so that it is possible to provide a structure favorable for enlarging the display area of a display.

According to an aspect of the present disclosure, an electronic device is provided that is capable of providing a structure applicable to all resistance-measurement-type biometric sensing.

According to an aspect of the present disclosure, an electronic device is provided in which a transparent electrode is formed on at least a portion of the surface of a transparent member so that visibility/transparency can be improved.

According to an aspect of the present disclosure, an electronic device is provided in which a transparent electrode formed on a display region is electrically connected to a main printed circuit board using a metal material on a side face of a transparent member.

According to an aspect of the present disclosure, an electronic device is provided in which a metallic material is used on a side surface of a transparent member such that a separate FPCB is not required, and thus a waterproof structure is not required.

According to an aspect of the present disclosure, an electronic device is provided that can be used for sensing biometric information using a resistance scheme.

In accordance with an aspect of the present disclosure, an electronic device includes a housing; a display panel at least partially accommodated in the housing; a first electrode formed over at least a portion of the display panel to be visually transparent and to be exposed outside the electronic device; and a second electrode formed in at least a portion of one face of the housing below the display panel to be exposed outside the electronic device.

In accordance with an aspect of the present disclosure, an electronic device includes a housing; a display panel at least partially accommodated in the housing; a transparent member coupled to the housing to cover the display panel, thereby forming one face of the electronic device; a substantially transparent first electrode formed on a face of the transparent member, which faces outside of the electronic device; a second electrode formed on a face of the housing, which is opposite the face of the transparent member; and a printed circuit board accommodated in the housing, and having a control circuit disposed therein to be electrically connected to the first electrode and the second electrode. The control circuit may be set to measure a user's biometric information using an electrical signal acquired through the first electrode or the second electrode.

In accordance with an aspect of the present disclosure, an electronic device includes a housing forming at least a portion of an outer face of the electronic device; at least one sensor; a display panel accommodated in the housing; a transparent member covering at least a portion of the display panel, and forming another portion of the outer face of the electronic device; a first electrode region and a second electrode region, which are formed in at least a portion of the housing or the transparent member, which forms the outer face; and a processor. The processor is configured to receive a designated input for the electronic device using the at least one sensor; select at least one of the first electrode region and the second electrode region in response to the designated input; and detect a user's biometric information based on an electrical signal obtained through the at least one of the first electrode region and the second electrode region.

### BRIEF DESCRIPTION OF THE DRAWINGS

The above and other aspects, features, and advantages of the present disclosure will be more apparent from the following detailed description when taken in conjunction with the accompanying drawings, in which:
FIG. 1 is a perspective view illustrating the appearance of an electronic device, according to an embodiment of the present disclosure;
FIG. 2 is an exploded perspective view illustrating the internal configuration of the electronic device, according to an embodiment of the present disclosure;
FIG. 3 is a cross-sectional view schematically illustrating the internal configuration of the electronic device, according to an embodiment of the present disclosure;
FIG. 4 is a cross-sectional view schematically illustrating the interior of an electronic device capable of measuring biometric information, according to an embodiment of the present disclosure;
FIGS. 5A and 5B are cross-sectional views each illustrating the interior of an electronic device capable of measuring biometric information, according to embodiments of the present disclosure;
FIGS. 6 to 14 are cross-sectional views illustrating electronic devices, each of which includes various electrode structures capable of measuring biometric information, according to embodiments of the present disclosure;
FIG. 15 is a block diagram of an electronic device that provides a biometric measurement function, according to an embodiment of the present disclosure;
FIGS. 16A and 16B each illustrate an electronic device that provides a biometric measurement function, according to embodiments of the present disclosure;
FIG. 17 is a flowchart of an operation for a biometric measurement function in an electronic device, according to an embodiment of the present disclosure;
FIG. 18A illustrates an electronic device, which performs the operation of FIG. 17, according to an embodiment of the present disclosure;
FIGS. 18B to 18D are views for describing the operation of FIG. 17, according to embodiments of the present disclosure;
FIG. 19 is a flowchart of an operation related to power control in an electronic device that provides a biometric measurement function, according to an embodiment of the present disclosure;
FIG. 20 is a flowchart of an operation related to input or output through a display in an electronic device that provides a biometric measurement function, according to an embodiment of the present disclosure;
FIGS. 21A and 21B are views for describing the operation of FIG. 20, according to embodiments of the present disclosure;
FIG. 22 is a flowchart of an operation related to input or output through a display in an electronic device that provides a biometric measurement function, according to an embodiment of the present disclosure; and
FIGS. 23A and 23B are views illustrating an electronic device that provides a biometric measurement function, according to embodiments of the present disclosure.

### DETAILED DESCRIPTION

Embodiments of the present disclosure may be described with reference to accompanying drawings. The same or similar components may be designated by the same or similar reference numerals although they are illustrated in different drawings. Detailed descriptions of constructions or processes known in the art may be omitted to avoid obscuring the subject matter of the present disclosure

In this disclosure, the expressions "include", "may include" and other similar terms, may refer to the existence of a corresponding disclosed function, operation, or constituent element, and do not limit one or more additional functions, operations, or constituent elements. Further, "have", "has", "had", and other similar terms, merely denote a certain feature, numeral, step, operation, element, component, or a combination thereof, and do not exclude the existence or possibility of the addition of one or more other features, numerals, steps, operations, elements, components, or combinations thereof.

The expressions "or" or "at least one of A and/or B" includes any and all combinations of the words listed together. For example, the expressions "A or B" or "at least A and/or B" may include A, may include B, or may include both A and B.

In this disclosure, expressions including ordinal numbers, such as "first" and "second", may modify various elements. However, such elements are not limited by the above expressions. For example, the above expressions do not limit the sequence and/or importance of the elements. The above expressions are used merely for the purpose of distinguishing an element from the other elements. For example, a first user device and a second user device indicate different user devices, although both are user devices. Accordingly, a first element may be referred to as a second element, and a second element may be referred to as a first element, without departing from the scope of the embodiments of the present disclosure.

When an element is referred to as being "coupled" or "connected" to any other element, it should be understood that not only may the element be coupled or connected directly to the other element, but also a third element may be interposed between them. In contrast, when an element is referred to as being "directly coupled" or "directly connected" to any other element, it should be understood that no other element is interposed between the two elements.

The terms used in this disclosure describe one or more certain embodiments and are not intended to limit the scope of the present disclosure. Terms of a singular form may include plural forms as well, unless the context explicitly indicates otherwise. Further, all of the terms used herein, including technical and scientific terms, have the same meaning as commonly understood by those of ordinary skill in the art to which the present disclosure pertains. Terms, such as those defined in a generally used dictionary, are to be interpreted to have the same meanings as the customary meanings in the relevant field of art, and are not to be interpreted to have idealized or excessively formal meanings unless expressly so defined in the present disclosure.

An electronic device, according to the present disclosure, may include a communication function. For example, the electronic device may include at least one of a smartphone, a tablet personal computer, a mobile phone, a video phone, an electronic book (e-book) reader, a desktop personal computer, a laptop personal computer, a netbook computer, a personal digital assistant (PDA), a portable multimedia player (PMP), an MP3 player, a mobile medical appliance, a camera, and a wearable device (e.g. a head-mounted-device (HMD), electronic glasses, electronic clothes, an electronic bracelet, an electronic necklace, an electronic accessory, electronic tattoos, or a smartwatch).

The electronic device may also be a smart home appliance with a communication function, such as a television, a digital versatile disk (DVD) player, an audio player, a refrigerator, an air conditioner, a vacuum cleaner, an oven, a microwave oven, a washing machine, an air cleaner, a set-top box, a TV box (e.g., Samsung HomeSync™, Apple TV™, or Google TV™), a game console, an electronic dictionary, an electronic key, a camcorder, and an electronic photo frame.

The electronic device may also include at least one of various medical appliances (e.g., magnetic resonance angiography (MRA), magnetic resonance imaging (MRI), computed tomography (CT), and ultrasonic machines), navigation equipment, a global positioning system (GPS) receiver, an event data recorder (EDR), a flight data recorder (FDR), an automotive infotainment device, electronic equipment for ships (e.g., ship navigation equipment and a gyrocompass), avionics devices, security equipment, a vehicle head unit, an industrial or home robot, an automatic teller machine (ATM), and a point of sale (POS) device.

The electronic device may also include at least one of a part of furniture or a building/structure, an electronic board, an electronic signature receiving device, a projector, and various types of measuring instruments (e.g., water meters, electric meters, gas meters, and radio wave meters).

Further, the electronic device may be a flexible device.

The electronic device may also be a combination of one or more of the aforementioned devices. Further, it will be apparent to those of ordinary skill in the art that the electronic device, according to the present disclosure, is not limited to the aforementioned devices.

Hereinafter, the term "user" may indicate a person who uses an electronic device or may indicate a device (e.g., an artificial intelligence electronic device) that uses an electronic device.

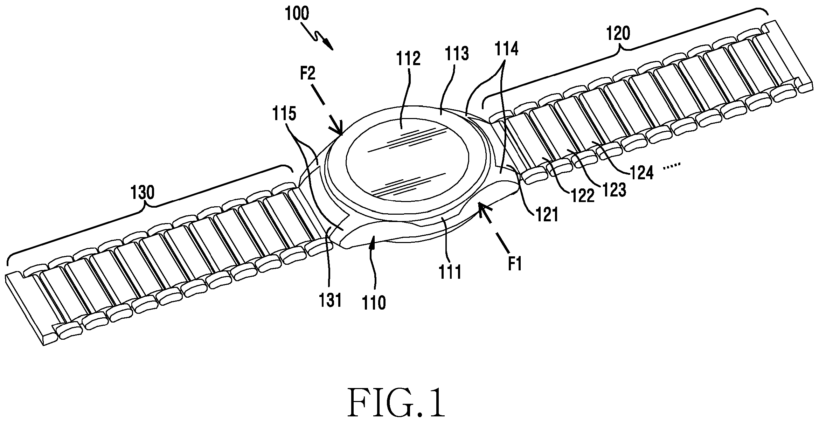

FIG. 1 is a perspective view illustrating an unfolding state of an electronic device mounted with a sensor module according to various embodiments of the present disclosure.

Referring to FIG. 1, an electronic device 100 is a wrist-wearable electronic device that is wearable on a user's wrist. The electronic device 100 includes a main body 110 and a pair of straps 120 and 130 (e.g., connection members, binding members, or chain members) mounted on opposite ends of the main body 110, respectively. The electronic device 100 may be worn on a user's wrist in a manner in which the pair of straps 120 and 130 is wound around the wrist while the main body 110 is placed on the wrist. One strap 120 may be provided with a buckle member, and the other strap 130 may be formed with a fastening member, to which the buckle member is fastened.

The main body 110 includes a housing 111. The housing 111 may be formed of a synthetic resin or a metal material. The main body 110 includes a display 112 disposed on the upper portion of the housing 111. The display 112 may be utilized as a touch screen device including a touch sensor and may include a pressure sensor configured to sense pressure applied to an exposed face of the display. The main body 110 includes an annular member 113 that is rotatably disposed in a manner that encloses the display 112. The annular member 113 may be installed in a manner that encloses at least a portion of the display 112 disposed in the housing 111. For example, the annular member may be referred to as a rotary wheel or an input wheel.

When the electronic device 100 is a wrist-wearable electronic device, the annular member may be disposed in a rotatable-bezel manner. According to an embodiment of the present disclosure, the annular member 113 may be rotated clockwise or counterclockwise, and the rotation amount may be limited by up to 360 degrees or may be configured to rotate infinitely. The electronic device 100 may detect rotation parameters (e.g., a rotation direction, a rotation speed, and a rotation amount) of the annular member 113, and may perform a corresponding function based on the detected parameters.

The housing 111 may include at least one key button. A battery (e.g., a rechargeable battery) may be applied to the electronic device 100 as a power supply unit within the electronic device 100, and a wireless charging coil member may be disposed in the electronic device 100 in order to charge the battery. The electronic device 100 may include at least one antenna device for communication. The antenna device may have at least one conductive pattern (e.g., antenna radiation pattern) arranged inside the electronic device 100. The electronic device 100 may be implemented to be selectively mounted on a predetermined portable charging cradle (e.g., a wired or wireless charging cradle) in order to charge the battery.

The housing 111 may include at least one sensor device disposed in at least a partial region of the housing 111. The sensor device may include at least one of a camera sensor, a fingerprint recognition sensor, an infrared sensor, an HRM(Heart Rate Measurement)sensor, an ultrasonic sensor, a photosensor, a proximity sensor, an illuminance sensor, a temperature sensor, and an iris recognition sensor.

The pair of straps 120 and 130 include a first strap 120 and a second strap 130 and may be formed of a metal material. The first strap 120 is fixed to a first strap fastening portion 114 on the housing 111, and the second strap 130 is fixed to a second strap fastening portion 115 on the housing 111.

The first strap 120 includes a plurality of unit links (e.g., the unit links 121, 122, 123, and 124) connected to each other to be rotatable with respect to each other and each having a predetermined length so as to define the length of the first strap 120. The unit links may be formed of a metal material, and may have the same length or different lengths. A single unit link 121, among the plurality of unit links, disposed at one end may serve as a first coupling member 121, which is fastened to the first strap fastening portion on the housing 111. The first coupling member 121 may be fastened to the first strap fastening portion 114 on the housing by a hinge pin. The second strap 130 also includes a plurality of unit links by which the length of the second strap is formed like the first strap 120, and among the plurality of unit links, the unit link disposed at one end serves as a second coupling member 131, which may be fastened to the second strap fastening portion 115 on the housing 111.

The electronic device 100 may be formed of a metal material for the purpose of reinforcing the rigidity and improving the design of the external appearance. In particular, the housing 111 formed of a metal material may be used as an antenna radiator by being electrically connected to a communication circuit disposed inside the housing in at least one region. According to an embodiment of the present disclosure, the housing 111 may be used as a multi-band antenna radiator that operates in different frequency bands by being electrically connected to the communication circuit at different power feeding positions F1 and F2.

The electronic device 100 may be degraded in radiation characteristics by the metal straps 120 and 130, which are in physical contact with the housing 111 when the housing 111 is used as an antenna radiator. According to an embodiment of the present disclosure, the metal straps 120 and 130 operate as undesired conductors of the metal housing 111, which is used as an antenna radiator, and as a result, the metal straps 120 and 130 operate as undesired elements, by which the radiation direction of current is distorted or the intensity of current is lowered. Thus, the metal straps 120 and 130 may operate as radiation-inhibiting elements of the antenna radiator. This is a problem that may be solved by applying an insulating structure between the metal housing 111 and the metal straps 120 and 130 in the electronic device 100.

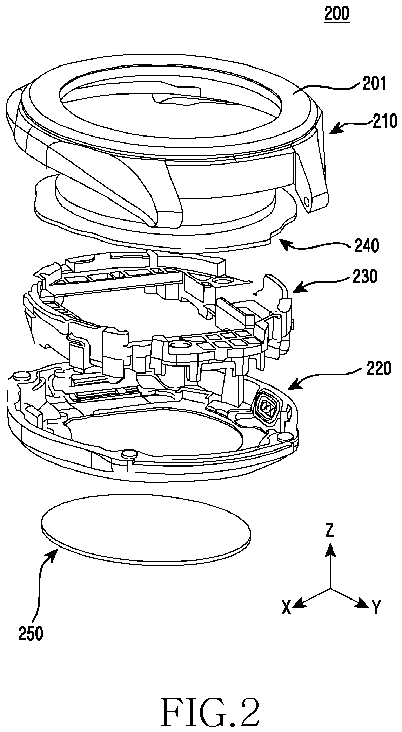

FIG. 2 is an exploded perspective view illustrating the internal configuration of the electronic device, according to an embodiment of the present disclosure.

Referring to FIG. 2, the electronic device 200 may be a wearable electronic device, at least a portion of which is the same as the electronic device 100 illustrated in FIG. 1. The wearable electronic device 200 includes a display 240, a housing 210 and 220, and a cover 250, which are associated with the appearance, and may include a support structure 230, which is accommodated in the housings 210 and 220 and supports various components.

Various electronic components in the electronic device 200 are fixed in the state of being supported on the support structure 230, and the support structure 230 is received in the housing 210 and 220. A first appearance component (e.g., the window of the display 240) is mounted on one face of the housings 210 and 220 and a second appearance component (e.g., the cover 250) may be coupled to the other face of the housing 210 and 220.

The housing 210 and 220 includes a front case 210 and a rear case 220. The front case 210 and the rear case 220 may be coupled to each other in the vertical direction, thereby forming a single housing.

The housing 210 and 220 may form the external appearance of the electronic device 200, may protect a plurality of components, and may be at least partially made of a metal material so as to perform an antenna radiator function. The assembly of the electronic device 200 may be completed by vertically coupling the support structure 230 on which various components are mounted to the housing 220 and vertically coupling the display 240 to the housing 220 to which the support structure 230 is coupled.

The support structure 230 may be a support bracket, a support member, or an inner support, and may support a plurality of electronic components. The support structure 230 may be disposed within the electronic device 200, and may be used as a component for strengthening the overall rigidity of the electronic device. For example, at least one of aluminum, magnesium, and STS(Stainless SUS) may be used for the support structure 230. A high-rigidity plastic containing glass fiber may be used for the support structure 230, or a metal and a plastic may be used together. When a metal and a non-metal material are used together as the material of the support structure 230, the support structure 230 may be formed by insert injection molding the non-metal material on the metal material.

Sheets (i.e., an elastic member, such as sponge or rubber, and an adhesive layer, such as double-sided tape or single-sided tape) may be additionally disposed between the display 240 and the front case 210 so as to protect the display 240.

A plurality of support structures 230 may be configured, in which a first structure supports the display 240 and a printed circuit board, and a second structure supports other members. For example, the second structure may be configured to support and protect a battery.

The rear cover 250 is a member that is disposed on the second face of the housing 220 so as to be exposed, and may be made of a synthetic resin material or a glass material. For example, the rear cover 250 may be made of a transparent material or a translucent or opaque material.

The front case 210 of the housing includes a rotary wheel 201 on the outer face thereof. The rotary wheel 201 may be an input device that is rotated in order to input desired data.

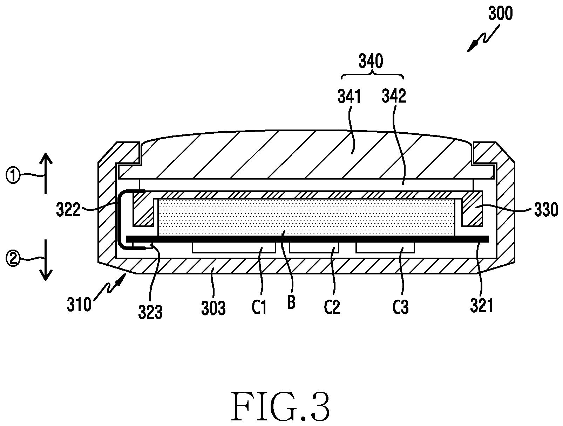

FIG. 3 is a cross-sectional view illustrating the internal configuration of the wearable electronic device, according to an embodiment of the present disclosure.

Referring to FIG. 3, the electronic device 300 includes a housing 310. The housing 310 may include a display 340 disposed on the first face thereof, which faces a first direction, and a rear cover 303 exposed on the second face thereof, which faces a second direction. A transparent member (e.g., a glass cover) 341 may be coupled to the housing 310, and a rear cover 303 may be coupled to the housing 310.

A display 340 may be supported on one face of the support structure 330, which faces the first direction, and a battery B and a printed circuit board 321 may be supported on the other face of the support structure 330, which faces the second direction.

The printed circuit board 321 may be disposed between the battery B, which is positioned to face the first direction, and the rear cover 303, which is positioned to face the second direction. Various electronic components C1, C2, and C3 may be mounted on the one face and the other face of the printed circuit board 321.

According to an embodiment of the present disclosure, in the electronic device 300, the display 340, the support structure 330, the battery B, the printed circuit board 321, and the back cover 303 are disposed to be sequentially stacked in the housing 310. However, the stacked structure in the electronic device is not limited thereto, and may be changed.

In the electronic device 300, the display 340 and the printed circuit board 321 may be electrically connected to each other by an electrical connection device 322. The electrical connection device 322 may include an FPCB, may be referred to as a display FPCB, and may be electrically connected to the printed circuit board 321 by a slim connector 323.

The display 340 includes a transparent member 341 and a display module 342, and only the transparent member 341 may be disposed so as to be exposed and thus constitute the appearance. The transparent member 341 may be made of a transparent synthetic resin or a glass material. The display module 342 may include a touch-sensitive panel (TSP). In this case, the display module 342 may form a touch screen.

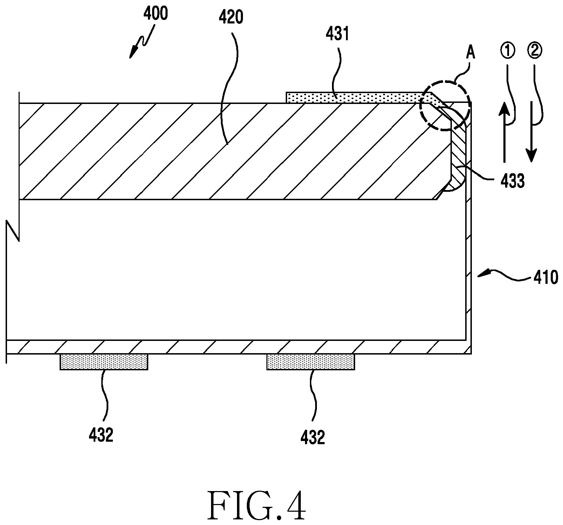

FIG. 4 is a cross-sectional view schematically illustrating the interior of an electronic device capable of measuring biometric information, according to an embodiment of the present disclosure;
Referring to FIG. 4, an electronic device 400 is capable of measuring biometric information, and may include a wearable-type electronic device, which is removable from the human body. The wearable-type electronic device 400 may be a watch-type or wrist-type wearable device, which is worn on the wrist.

The electronic device 400 may perform a bioelectrical impedance analysis (BIA) on a person's body, may perform an electrocardiogram (ECG), or may measure a galvanic skin response (GSR).

The BIA is a method of passing a weak electric current through a body, measuring the amount of water in the body using electric resistance, and calculating the amount of fat by converting the amount of water into the amount of fat. The BIA generates micro-current in two electrodes, which are in contact with one hand (in practice, two or more electrodes may be used in order to improve measurement accuracy), and a resistance value may be measured in two electrodes placed in the other hand (in practice, two or more electrodes may be used in order to improve measurement accuracy).

The ECG is a measurement method that is capable of measuring a minute action potential difference (e.g., 1 mV voltage) generated on the myocardium of the heart when the heart is pulsed with electrodes attached to a person's body surface. The ECG usually uses two or more electrodes, in which one electrode grounded to the left hand, one electrode grounded to the right hand, and one electrode used as a ground may be used. The ECG may use two or more electrodes without a ground electrode, or may use one or more electrodes for the purpose of increasing the accuracy of a measured value.

The electrical resistance GSR may be temporarily reduced or an action potential may be temporarily generated due to electrical skin reflex, that is, external stimuli or emotional excitation in the skin of a person's body. The GSR may be measured by a method of bringing an electrode into contact with the skin and capturing, amplifying, and recording a change in electrical resistance or action potential. The GSR may be measured in one hand using two electrodes. Three filter electrodes may be used in order to improve the accuracy of measurement of the GSR.

The electronic device 400 includes a housing 410 that forms an appearance and protects various electronic components mounted therein. The housing 410 may include a first face, which faces the first direction, a second face, which faces the second direction, and a third face, which faces a third direction perpendicular to each of the first and second directions, and encloses at least a portion of a space between the first and second faces, so long as the housing 410 has a polygonal shape (e.g., a rectangular shape) when viewed from above. When the first direction is a direction facing upward, the first face may be the front face of the housing 410, when the second direction is a direction facing downward, the second face may be the rear face of the housing 410, and when the third direction is a direction facing a lateral side, the third face may be a side face. The side face may include a plurality of side faces. For example, the plurality of side faces may be oriented in the transverse or longitudinal direction of the housing 410 and may include a side face at the top edge, bottom edge, left edge, and right edge of the housing 410. The housing 410 may be constituted by one case, or may be constituted by coupling front and rear cases to each other.

The housing 410 includes a transparent member 420 formed to at least partially overlap the housing. The transparent member 420 may be exposed on at least a portion of the first face of the housing 410, and may form at least a portion of the first face of the housing 410. The transparent member 420 may be referred to as a transparent window, a transparent plate, or a front window. Further, the transparent member 420 may be a display module. The transparent member 420 may be made of a synthetic resin such as a polycarbonate (PC) material or acryl, or a glass material and may be formed in a curved shape having a curvature.

The transparent member 420 may be disposed parallel to the first face of the housing 410. In addition, the transparent member 420 may be made of a rigid material or a flexible material.

The electronic device 400 may include at least one first electrode 431 (or a first electrode region) and at least one second electrode 432 (or a second electrode region 432) in order to acquire biometric information. Each of the first electrodes 431 and the second electrode 432 may be electrically in contact with at least a portion of a person's body as a conductive member or a conductive region.

The first electrode 431 may be visually transparent above at least a portion of the display panel. In addition, the first electrode 431 may be formed to be exposed outside the electronic device 400. The first electrode 431 may be formed to be in contact with at least a portion of the first face of the housing 410. The first electrode 431 may be electrically connected to at least a portion of the person's body. The first electrode 431 may be arranged to overlap at least a portion of the display when viewed from above the first electrode.

The first electrode 431 may be made of a substantially transparent material and may be disposed on at least a portion of the transparent member 420 so as to be visually transparent. For example, the first electrode 431 may be formed by being coated with any one of indium tin oxide (ITO), AZO(azo compound), and SnO(Stannum Oxide). The first electrode 431 may be coated by a sputtering process, a CVD(Chemical Vapor Deposition) process, or a PVD(Physical Vapor Deposition) process. In addition, the first electrode 431 is a transparent electrode, and may be made of an electrochromic material such that the first electrode 431 can be colored only when measuring biometric information. For example, the electrochromic material may include any one of WO₃, Nb₂O₅, MoO₃, TiO₃ (cathodic coloration), V₂O₅, IrO₂, and NiO (anodic coloration).

In addition, the first electrode 431 may be disposed along at least a portion of a rim region or along at least a portion of an edge region of the transparent member 420. The first electrode 431 may be formed in the shape of a layer having a thickness on the first face of the transparent member 420, which faces the first direction. In addition, a plurality of the first electrodes 431 may be disposed on one face of the transparent member 420 to have a predetermined shape and to be spaced apart from each other. The first direction refers to the direction in which the transparent member is exposed to the outside, and the second direction may be opposite the first direction.

The second electrode 432 may be disposed on at least a portion of the second face of the housing 420, and may be electrically connected to at least a portion of the person's body. At least one second electrode 432 may be disposed on the rear face of the housing 410. The second electrode 432 may be formed in at least a portion of one face of the housing 410 under the display panel so as to be exposed outside the electronic device 400.

The second electrode 432 may be made of a transparent material or an opaque material. For example, the second electrode 432 may be formed by being inserted into or coated on at least a portion of the second face using any one of Aluminum, copper, platinum, and gold. The second electrode 432 may be inserted into the second face using an adhesive to attach the second electrode 432 or may be formed integrally with the second face through insert injection molding when the second face is injection molded. The coating of the second electrode 432 may be formed by a sputtering process, a CVD process, a PVD process, or an electroplating process.

According to the above configuration, the electronic device 400 may be configured such that the first electrode 431 and the second electrode 432 are set to be electrically connected to each other when the first electrode 431 is attached to the first body portion of the user of the electronic device and the second electrode 432 is attached to the second body portion of the user of the electronic device.

The electronic device 400 may further include a conductive portion 433 disposed on a side face of the transparent member 420. The conductive portion 433 may be at least partially disposed on a portion of the transparent member 420 and at least part of the conductive portion 433 may be electrically connected to the first electrode 431. The conductive portion 433 may be formed in the shape of a layer having a given thickness on a side face of the transparent member. The conductive portion 433 may be made of a transparent material or an opaque material including a conductive material. For example, the conductive portion may be made of any one of a metal material, an ITO material, and a PEDOT(3,4-ethylenedioxythiophene) material. The conductive portion 433 may be a metal through which a measured biometric signal is transmitted to the first electrode 431. The conductive portion 433 is not limited to being disposed on the side face of the transparent member 420, and may be disposed on the first face or the second face of the transparent member 420 rather than on the side face of the transparent member 420 so long as the conductive portion 433 is disposed so as to overlap the first electrode 431. The conductive portion may be referred to as a conductive layer.

The transparent member 420 may have at least one chamfered portion formed on at least a portion of each corner. The chamfered portion may extend along the corner of the transparent member 420. In addition, the chamfered portion may be configured such that at least a portion of the first electrode 431 and at least an end portion of the conductive portion 433 may be arranged to overlap each other so as to form an electrically conductive structure therebetween. The chamfered portion may be formed at each of an upper-end corner and a lower-end corner of the transparent member 420. The overlapping portion may be located at the upper-end corner so that an electrically conductive structure of the first electrode 431 and the conductive portion 433 are disposed on the chamfered portion of the upper-end corner.

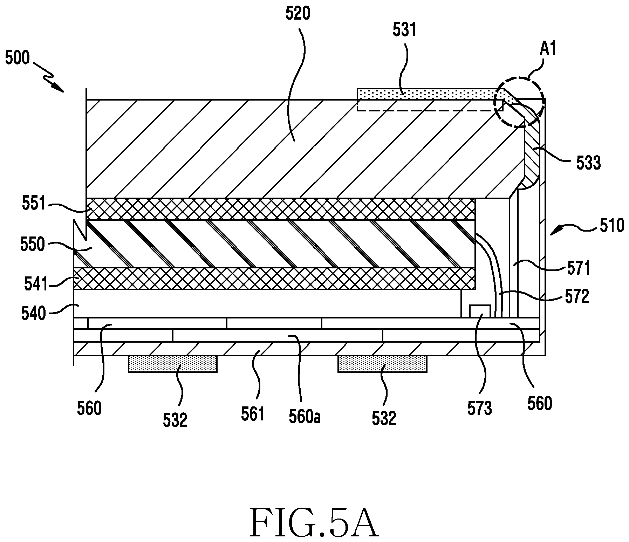

FIG. 5A is cross-sectional view illustrating the interior of an electronic device capable of measuring biometric information, according to an embodiment of the present disclosure.

Referring to FIG. 5A, an electronic device 500 includes a housing 510. Since the housing 510 is at least partially or entirely the same as the housing 410 illustrated in FIG. 4, detailed descriptions thereof will be omitted. In addition, in the electronic device 500, the first electrode 531, the second electrode 532, and the conductive portion 533 may be configured to be at least partially or entirely the same as the first electrode 431, the second electrode 432, and the conductive portion 433 illustrated in FIG. 4. Thus, detailed descriptions thereof will be omitted. In addition, the conductive structure of the first electrode 531 and the conductive portion 533 formed in the chamfered portion A1 of the transparent member 520 (e.g., the glass cover) is configured to be at least partially or entirely the same as the conductive structure of the first and electrode 431 and the conductive portion 433 provided in the chamfered portion A illustrated in FIG. 4. Thus, detailed descriptions thereof will be omitted.

The electronic device 500 includes a transparent member 520, a touch panel 550 (e.g., a touch film) coupled to the transparent member 520 by a first transparent bonding portion 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520. In the display panel 540, a first portion, above which the first electrode 531 is located, may perform biometric measurement, and a second portion, other than the first portion, may provide a display region.

The part indicated by reference numeral 560a may be an auxiliary printed circuit board.

The transparent member 520 may be disposed between the display panel 540 and the first electrode 531. The first electrode 531 may be formed on at least a portion of the surface of the transparent member 520 facing the outside.

The display panel 540 may be disposed between the first and second faces of the housing 510, and may be disposed such that at least a portion of the display panel 540 is exposed to the outside through the transparent member 520. The first electrode 531 may be disposed to at least partially overlap the display panel 540 (including the touch panel) when viewed from above the first face of the housing 510 (in a top plan view).

The electronic device 500 may have the conductive portion 533 electrically connected to the printed circuit board 560 by a first electrical connection device 571. The first electrical connection device 571 may include an FPCB, a pogo pin, or a metal. The electronic device 500 may be electrically connected to the printed circuit board 560 by a second electrical connection device 572 which may include an FPCB or the like.

The second electrode 532 may be electrically connected to a control integrated circuit (IC) (e.g., a control circuit) 573 disposed on the printed circuit board 560. The electronic device 500 may measure the user's biometric information (e.g., heartbeat information, body fat information, body water information, or stress information) using at least one first electrode 531 or at least one second electrode 532 under the control of the control IC (e.g., a sensing circuit) 573 mounted on the printed circuit board 560. The first and second electrodes 531 and 532 are electrodes electrically connected to a person's body, while the conductive portion 533 may serve as a conductive member that electrically connects the first electrode 531 to the printed circuit board 560 or the control IC 573.

For example, when the electronic device 500 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

A portion of the first electrode 531 may fill a portion of the recess (dotted line portion) formed in the transparent substrate 520.

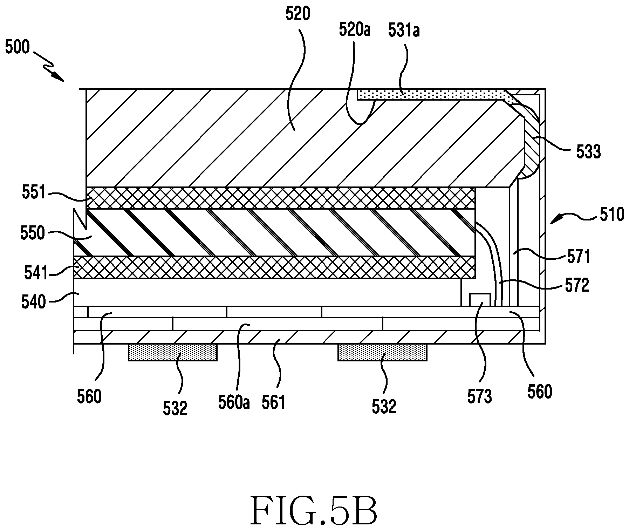

FIG. 5B is cross-sectional view illustrating the interior of another electronic device capable of measuring biometric information, according to an embodiment of the present disclosure.

Referring to FIG. 5B, the first electrode 531a may be disposed to be substantially coplanar to the first face of the transparent substrate 520. For example, the transparent substrate 520 may be configured such that a recess 520a is formed in the first face thereof, and the first electrode 531a is formed in the recess 520a. For example, in order to minimize the protrusion of the first electrode 531a from the first face of the transparent electrode 520, the first face of the transparent electrode 520 and one face of the first electrode 531a may be formed in a coplanar shape, for example, a substantially seamless shape that does not have a boundary line.

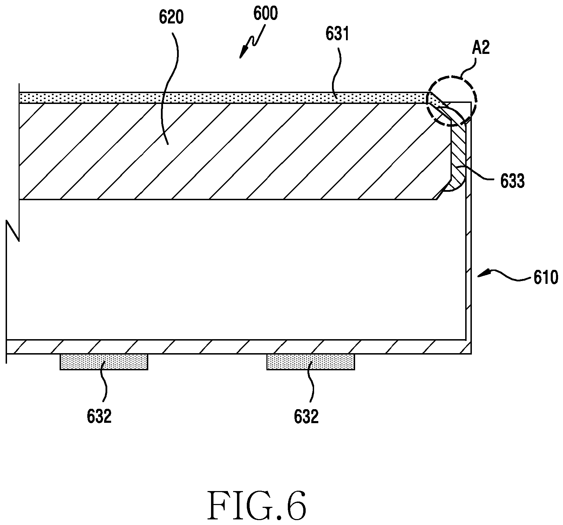

FIGS. 6 to 14 are cross-sectional views respectively illustrating electronic devices, each of which includes various electrode structures capable of measuring biometric information, according to various embodiments of the present disclosure.

Referring to FIG. 6, an electronic device 600 is at least partially or entirely the same as the electronic device 400 illustrated in FIG. 4, and may be configured such that the configuration only of a first electrode 631 is different from that of the electronic device 400 shown in FIG. 4.

The first electrode 631 may be formed entirely on the face of the transparent member 620, which faces the first direction, and may be formed to have a width to cover the transparent member 620 when viewed from above the first face of the housing 610. The first electrode 631 may be formed of a substantially visually transparent material in the shape of a layer. For example, the transparent member 620 may be at least a portion of a display.

The first electrode 631 is a transparent electrode that is disposed to overlap the transparent member 620 in the vertical direction, and to overlap the second face of the housing 610 in the vertical direction. For example, at least a portion of the first electrode 631 may be arranged to overlap the second electrode 632 in the vertical direction.

The second electrode 632 may be disposed on at least a portion of the second face of the housing 610, and may be electrically connected to at least a portion of the person's body. At least one second electrode 632 may be disposed on the rear face of the housing 610. The second electrode 632 may be made of a transparent material or an opaque material. For example, the second electrode 632 may be formed by coating any one of aluminum, copper, platinum and gold on at least a portion of the second face. The coating of the second electrode 632 may be formed through a sputtering process, a CVD process, a PVD process, or an electroplating process.

The electronic device 600 includes a conductive portion 633 disposed on a side face of the transparent member 620. The conductive portion 633 may be at least partially disposed on a portion of the transparent member 620 and at least part of the conductive portion 633 may be electrically connected to the first electrode 631. The conductive portion 633 may be formed in the shape of a layer having a thickness on a side face of the transparent member. The conductive portion 633 may be made of a transparent material or an opaque material that may include a conductive material. For example, the conductive portion 633 may be made of any one of a metal material, an ITO material, and a PEDOT material.

The transparent member 620 may have at least one chamfered portion A2 formed on at least a portion of each corner. The chamfered portion A2 may extend along the corner of the transparent member 620 and may be configured such that at least a portion of the first electrode 631 and at least a portion of the conductive portion 633 are arranged to overlap each other so as to form an electrically conductive structure therebetween. The chamfered portion A2 may be formed at each of an upper-end corner and a lower-end corner of the transparent member 620. The overlapping portion may be located at the upper-end corner so that an electrically conductive structure of the first and third electrodes 631 and 633 may be disposed on the chamfered portion A2 of the upper-end corner.

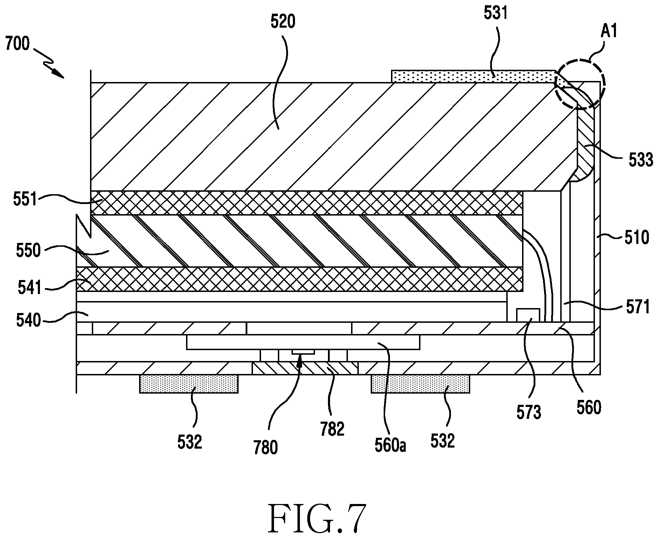

Referring to FIG. 7, an electronic device 700 may be configured to be the same as the electronic device 500 illustrated in FIG. 5, except that an illuminance sensor 780 and a transparent window 782 are additionally provided.

Referring to FIG. 7, the electronic device 700 includes a housing 510. Since the housing 510 is at least partially or entirely the same as the housing 410 illustrated in FIG. 4, detailed descriptions thereof will be omitted. In addition, in the electronic device 700 according to various embodiments, the first electrode 531, the second electrode 532, and the conductive portion 533 may be configured to be at least partially or entirely the same as the first electrode 431, the second electrode 432, and the conductive portion 433 illustrated in FIG. 4. Thus, detailed descriptions thereof will be omitted. In addition, the conductive structure of the first electrodes 531 and the conductive portion 533 formed in the chamfered portion A1 of the transparent member 520 (e.g., the glass cover) is configured to be at least partially or entirely the same as the conductive structure of the first electrode 431 and the conductive portion 433 provided in the chamfered portion A illustrated in FIG. 4. Thus, detailed descriptions thereof will be omitted.

The electronic device 700 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent member 520. The part indicated by reference numeral 560a may be an auxiliary printed circuit board.

The display panel 540 may be disposed between the first and second faces of the housing 510, and may be disposed such that at least a portion of the display panel 540 is exposed to the outside through the transparent member 520. The first electrode 531 may be disposed to at least partially overlap the display panel 540 (including the touch panel) when viewed from above the first face of the housing 510 (in a top plan view).

The conductive portion 533 of the electronic device 700 may be electrically connected to the printed circuit board 560 by a first electrical connection device 571. For example, the first electrical connection device 571 may include an FPCB. The touch panel 550 may be electrically connected to the printed circuit board 560 by a second electrical connection device 572. For example, the second electrical connection device 572 may also include an FPCB.

The second electrode 532 may be electrically connected to a control IC (a control circuit) 573 disposed on the printed circuit board 560. The electronic device 700 may measure the user's biometric information (e.g., heartbeat information, body fat information, body water information, or stress information) using at least one first electrode 531 or at least one second electrode 532 under the control of the control IC (e.g., a sensing circuit) 573 mounted on the printed circuit board 560. The first and second electrodes 531 and 532 are electrodes electrically connected to a person's body, while the conductive portion 533 may serve as a conductive member that electrically connects the first electrode 531 to the printed circuit board 560 or the control IC 573.

For example, when the electronic device 700 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

In the electronic device 700, the illuminance sensor 780 is disposed in the second face of the housing 510. The illuminance sensor 780 may be mounted on the face of the printed circuit board 560a, which faces the second direction, and may be disposed to be exposed outside the housing 510. A transparent window 782 may be configured on the second face of the housing 510 to face the illuminance sensor 780.

One or more second electrodes 532 may be disposed around the illuminance sensor 780. For example, the second electrodes 532 may be disposed symmetrically about the illuminance sensor 780, or may be opposed to each other.

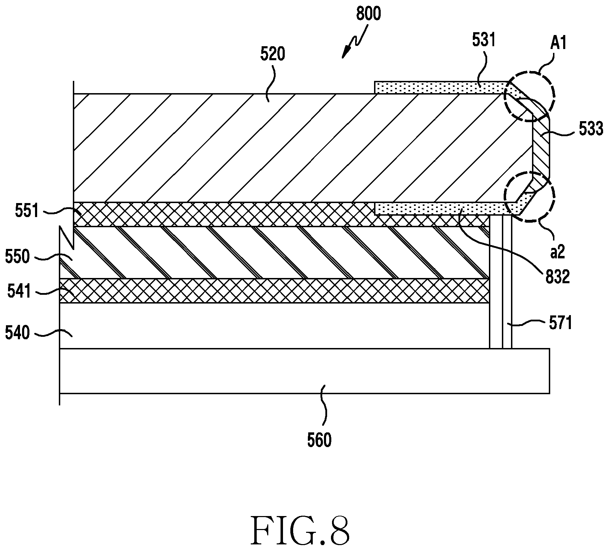

Referring to FIG. 8, the first electrode 531, the second electrode 832, and the conductive portion 533 may be configured in the electronic device 800 to be at least partially or entirely the same as the first electrode 431, the second electrode 432, and the conductive portion 433 illustrated in FIG. 4. In addition, the conductive structure of the first electrodes 531 and the conductive portion 533 formed in the chamfered portion A1 of the transparent member 520 (e.g., the glass cover) may be configured to be at least partially or entirely the same as the conductive structure of the first electrode 431 and the conductive portion 433 provided in the chamfered portion A illustrated in FIG. 4. A detailed description of similar components is omitted.

The electronic device 800 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520.

The display panel 540 may be disposed such that at least a portion of the display panel 540 is exposed to the outside through the transparent member 520. The first electrode 531 may be disposed to at least partially overlap the display panel 540 (including the touch panel).

The conductive portion 533 of the electronic device 800 may be electrically connected to the printed circuit board 560 by a first electrical connection device 571. For example, the first electrical connection device 571 may include an FPCB.

The electronic device 800 may measure the user's biometric information (e.g., heartbeat information, body fat information, body water information, or stress information) using at least one first electrode 531 or at least one second electrode 832 under the control of the control IC mounted on the printed circuit board 560. The first and second electrodes 531 and 832 are electrodes electrically connected to a person's body, while the conductive portion 533 may serve as a conductive member that electrically connects the first electrode 531 to the printed circuit board 560 or the control IC.

For example, when the electronic device 800 is a wearable electronic device worn on the wrist, the first electrode 531 may be touched by and electrically connected to a portion of a person's body (e.g., a finger), and the second electrode 832 may be touched by and electrically connected to a portion of a person's body (e.g., the skin on a wrist).

The first electrode 531 is disposed on at least a portion of one face of the transparent member 520, which faces the first direction, and the second electrode 832 may be disposed on at least a portion of the other face of the transparent member 520, which faces the second direction.

The first electrode 531 may be configured to be at least partially or entirely the same as the first electrode 431 illustrated in FIG. 4.

The second electrode 832 may be opposed to the first electrode 531 and may be arranged to overlap at least a portion of the transparent member 520 and to at least partially overlap the first electrode 531. In addition, the second electrode 832 may be arranged to overlap at least a portion of the transparent member 520 when viewed from above the transparent member 520 in the first direction.

The second electrode 832 may be made of a substantially transparent material. The second electrode 832 is a transparent substrate, and may be disposed on the substantially transparent member 520 or on the display panel 540 (including the touch panel 550). For example, the second electrode 832 may be formed by being coated with any one of ITO, AZO, and SnO. The second electrode 832 may be coated through a sputtering process, a CVD process, a PVD process, or the like.

In addition, the second electrode 832 may be disposed along at least a portion of a rim region or along at least a portion of an edge region of the transparent member 520. The second electrode 832 may be formed in the shape of a layer on the other face of the transparent member 520, which faces the second direction.

The second electrode 832 may be electrically connected to the printed circuit board 560 or a control circuit included in the printed circuit board 560 using the electrical connection member 571. The electrical connection member 571 may include an FPCB, a pogo pin, or a metal.

The electronic device 800 may further include a conductive portion 533 electrically connected to the first and second electrodes 531 and 832. The conductive portion 833 may be conductive with at least a portion of the first electrode 531 in the upper-end chamfered portion A1, and conductive with at least a portion of the second electrode 832 in the lower-end chamfered portion a2. The upper-end chamfered portion A1 or the lower-end chamfered portion a2 may be configured in an overlapping conductive structure. For example, in the upper-end chamfered portion A1, a first conductive structure, in which at least a portion of the first electrode 531 and at least a portion of the conductive portion 533 overlap each other, may be provided. In the lower-end chamfered portion a2, a second conductive structure, in which at least a portion of the second electrode 832 and at least a portion of the conductive portion 533 overlap each other, may be provided.

An electrical path enables a biometric signal to be transmitted to a control circuit in the printed circuit board 560 via the first electrode 531, the conductive portion 533, the second electrode 832, and the electrical connecting member 871, which are made conductive by being touched with a finger.

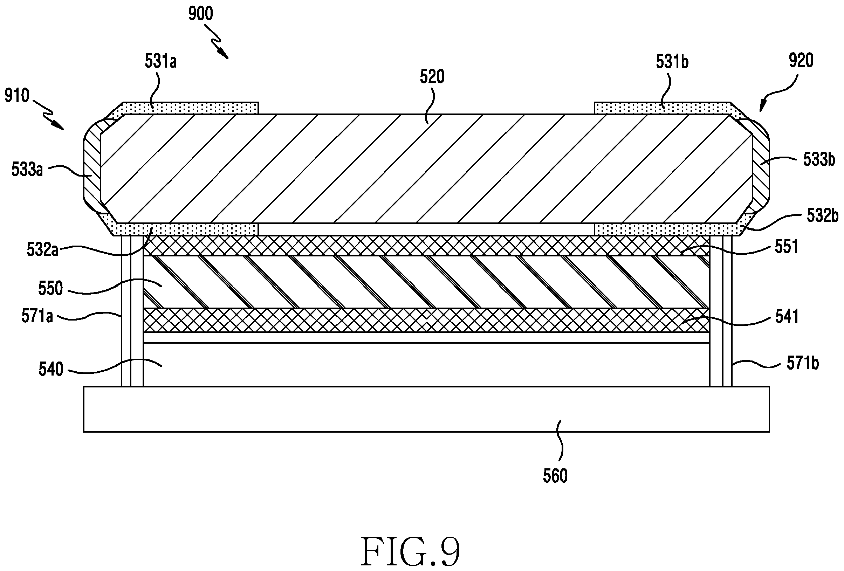

FIG. 9 is a cross-sectional view illustrating an electronic device, which includes various electrode structures capable of measuring biometric information, according to an embodiment of the present disclosure.

Referring to FIG. 9, an electronic device 900 includes first and second electrodes 910 and 920, which constitute the electrode structures illustrated in FIG. 8, on both sides of the transparent member 520 in order to detect biometric information using two fingers. The first electrode structure 910 and the second electrode structure 920 may be symmetrically opposed to each other and may be configured to have the same structures as the electrode structures provided on the transparent member 520 illustrated in FIG. 8. The first and second electrode structures 910 and 920 may be symmetrically disposed at the left and right.

The first electrode structure 910 may be configured to be at least partially or entirely the same as the configuration of the first electrode 531, the second electrode 832, and the conductive portion 533 illustrated in FIG. 8. In addition, the conductive structure of the first electrode 531 and the conductive portion 533 formed in the chamfered portion A1 of the transparent member 520 (e.g., the glass cover) may be configured to be at least partially or entirely the same as the conductive structure of the first electrode 431 and the conductive portion 433 provided in the chamfered portion A illustrated in FIG. 4.

The electronic device 900 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520.

The electronic device 900 may have a second electrode 532a electrically connected to the printed circuit board 560 by a first electrical connection device 571a. For example, the first electrical connection device 571a may include an FPCB.

The second electrode 532a may be electrically connected to a control IC (e.g., control circuit) disposed on the printed circuit board 560. The electronic device 900 may measure the user's biometric information (e.g., heartbeat information, body fat information, body water information, or stress information) using at least one first electrode 531a or at least one second electrode 532a under the control of the control IC (e.g., sensing circuit) mounted on the printed circuit board 560. The first electrode 531a is an electrode electrically connected to a person's body, while the conductive portion 533a may serve as a conductive member that electrically connects the first electrode 531a to the printed circuit board 560 or the control IC.

The second electrode structure 920 may be configured to be at least partially or entirely the same as the configuration of the first electrode 531, the second electrode 832, and the conductive portion 533 illustrated in FIG. 8. In addition, the conductive structure of the first electrode 531a and conductive portion 533a, or the second electrode 532b and conductive portion 533b, which are respectively formed in the chamfered portions of the transparent member 520 (e.g., the glass cover), may be configured to be at least partially or entirely the same as the conductive structure of the first electrode 431 and the conductive portion 433 provided in the chamfered portion A illustrated in FIG. 4.

The electronic device 900 may have a second electrode 532b electrically connected to the printed circuit board 560 by a second electrical connection device 571b. The second electrical connection device 571b may include an FPCB.

The second electrode 532b may be electrically connected to a control IC (e.g., control circuit) disposed on the printed circuit board 560. The electronic device 900 may measure the user's biometric information (e.g., heartbeat information, body fat information, body water information, or stress information) using at least one first electrode 531b or at least one second electrode 532b under the control of the control IC (e.g., sensing circuit) mounted on the printed circuit board 560. The first electrode 531b is an electrode electrically connected to a person's body, while the conductive portion 533b may serve as a conductive member that electrically connects the first electrode 531b to the printed circuit board 560 or the control IC.

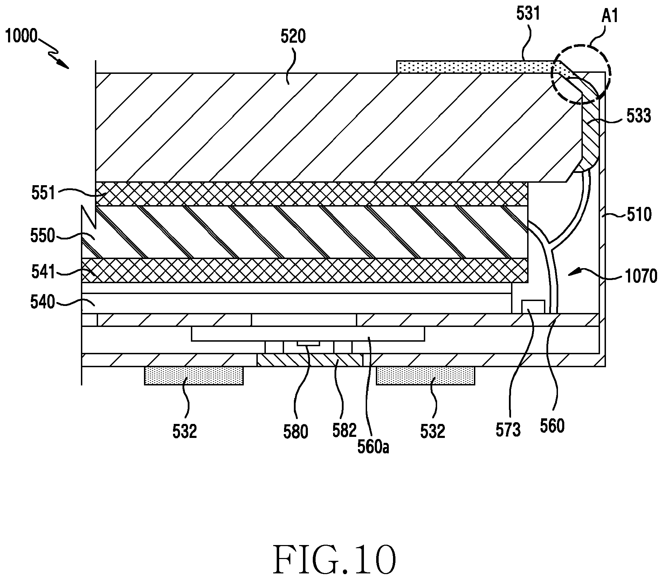

FIG. 10 is a cross-sectional view illustrating an electronic device, which includes various electrode structures capable of measuring biometric information, according to an embodiment of the present disclosure.

Referring to FIG. 10, an electronic device 1000 may be configured to be the same as the electronic device 700 illustrated in FIG. 7, except for an electrical connection member 1070.

The electronic device 1000 includes the housing 510. In addition, the configuration of the first electrode 531, the second electrode 532, and the conductive portion 533 may be configured to be at least partially or entirely the same as the configuration of the first electrode 431, the second electrode 432, and the conductive portion 433 illustrated in FIG. 4. In addition, the conductive structure of the first electrode 531 and the conductive portion 533 formed in the chamfered portion A1 of the transparent member 520 (e.g., the glass cover) may be configured to be at least partially or entirely the same as the conductive structure of the first electrode 431 and the conductive portion 433 provided in the chamfered portion A illustrated in FIG. 4.

The electronic device 1000 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520. The part indicated by reference numeral 560a may be an auxiliary printed circuit board.

The display panel 540 may be disposed between the first and second faces of the housing 510, such that at least a portion of the display panel 540 is exposed to the outside through the transparent member 520. The first electrode 531 may be disposed to at least partially overlap the display panel 540 (including the touch panel) when viewed from above the first face of the housing 510 (in a top plan view).

The electronic device 500 may have a conductive portion 533 electrically connected to the printed circuit board 560 by the electrical connection device 1070. The electronic device 1000 may include a touch panel 550, which may be electrically connected to the printed circuit board 560 by the electrical connection device 1070. For example, the electrical connection device 1070 may include an FPCB or the like.

The second electrode 532 may be electrically connected to a control IC (e.g., control circuit) disposed on the printed circuit board 560. The electronic device 1000 may measure the user's biometric information (e.g., heartbeat information, body fat information, body water information, or stress information) using at least one first electrode 531 or at least one second electrode 532 under the control of the control IC (e.g., sensing circuit) mounted on the printed circuit board 560. The first and second electrodes 531 and 532 are electrodes that are electrically connected to a person's body, while the conductive portion 533 serves as a conductive member that electrically connects the first electrode 531 to the printed circuit board 560 or the control IC.

When the electronic device 1000 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

The electronic device 1000 may use one electrical connection device (e.g., an FPCB 1070) rather than two electrical connection devices in order to electrically connect each of the conductive portion 533 and the touch panel 550 to the printed circuit board 560. The electrical connection device 1070 may be electrically connected at one end thereof to the printed circuit board 560 and may be bi-forked at the other end, in which one prong is electrically connected to the touch panel 550 and the other prong may be electrically connected to the conductive portion 533.

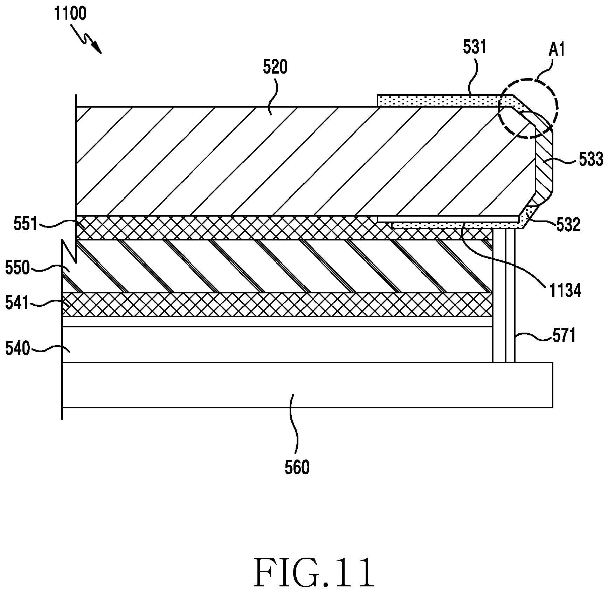

FIG. 11 is a cross-sectional view illustrating an electronic device, which includes various electrode structures capable of measuring biometric information, according to an embodiment of the present disclosure.

Referring to FIG. 11, an electronic device 1100 may be configured to be the same as the electronic device 800 illustrated in FIG. 8, except that a printed layer 1134 is formed on a transparent member 520.

In the electronic device 1100, the first electrode 531, the second electrode 532, and the conductive portion 533 may be configured to be at least partially or entirely the same as the first electrode 531, the second electrode 832, and the conductive portion 533 illustrated in FIG. 8. In addition, the conductive structure of the first electrode 531 and the conductive portion 533 formed in the chamfered portion A1 of the transparent member 520 (e.g., the glass cover) may be configured to be at least partially or entirely the same as the conductive structure of the first electrode 431 and the conductive portion 433 provided in the chamfered portion A illustrated in FIG. 4.

The electronic device 1100 may include a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive portion 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520. The part indicated by reference numeral 560a may be an auxiliary printed circuit board.

The display panel 540 may be disposed such that at least a portion of the display panel 540 is exposed to the outside through the transparent member 520. The first electrode 531 may be disposed to at least partially overlap the display panel 540 (including the touch panel).

The electronic device 1100 may have a second electrode 532 electrically connected to the printed circuit board 560 by the electrical connection device 571. For example, the electrical connection device 571 may include an FPCB.

The electronic device 1100 may measure the user's biometric information (e.g., heartbeat information, body fat information, body water information, or stress information) using at least one first electrode 531 or at least one second electrode 532 under the control of the control IC (e.g., a sensing circuit) mounted on the printed circuit board 560. The first and second electrodes 531 and 532 are electrodes electrically connected to a person's body, while the conductive portion 533 may serve as a conductive member that electrically connects the first electrode 531 to the printed circuit board 560 or the control IC.

For example, when the electronic device 1100 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

When the print layer 1134 is formed on at least a portion of the face of the transparent member 520, which faces the second direction, the second electrode 532 may be formed on the face of the print layer 1134, which faces the second direction. The print layer 1134 may be made of an opaque material, and the second electrode 532 may be configured as a transparent electrode or a metal electrode. The second electrode 532 may be formed by being laminated in the vertical direction with the print layer 1134.

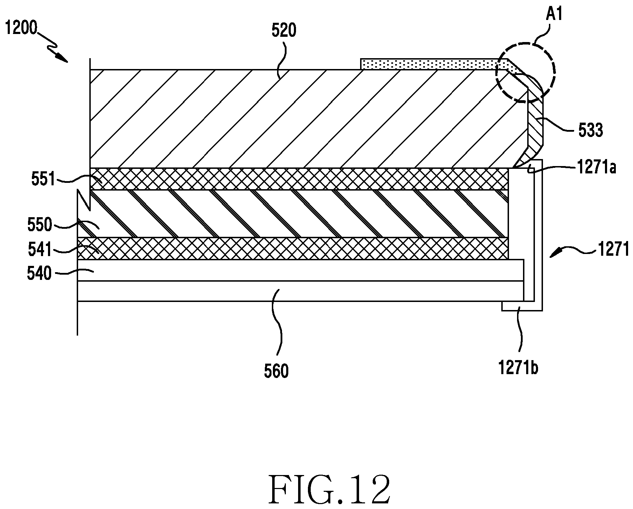

FIG. 12 is a cross-sectional view illustrating an electronic device, which includes various electrode structures capable of measuring biometric information, according to an embodiment of the present disclosure.

Referring to FIG. 12, an electronic device 1200 may be configured to be the same as the electronic device 500 illustrated in FIG. 5, except for the configuration of an electrical connection member 1271 that electrically connects the conductive portion 533 to the printed circuit board 560.

In the electronic device 1200, the first electrode 531 and the conductive portion 533 may be configured to be at least partially or entirely the same as the first electrode 531 and the conductive portion 533 illustrated in FIG. 5. In addition, the conductive structure of the first electrode 531 and the conductive portion 533 formed in the chamfered portion A1 of the transparent member 520 (e.g., the glass cover) may be configured to be at least partially or entirely the same as the conductive structure of the first electrode 531 and the conductive portion 533 provided in the chamfered portion A1 illustrated in FIG. 5.

The electronic device 1200 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520.

The display panel 540 may be disposed such that at least a portion of the display panel 540 is exposed to the outside through the transparent member 520. The first electrode 531 may be disposed to at least partially overlap the display panel 540 (including the touch panel).

In the electronic device 1200, the conductive portion 533 may be electrically connected to the printed circuit board 560 by a first electrical connection device 1271.

The electronic device 1200 may measure the user's biometric information (e.g., heartbeat information, body fat information, body water information, or stress information) using at least one first electrode 531 under the control of the control IC (e.g., a sensing circuit) mounted on the printed circuit board 560. The first electrode 531 is an electrode electrically connected to a person's body, while the conductive portion 533 may serve as a conductive member that electrically connects the first electrode 531 to the printed circuit board 560 or to the control IC.

For example, when the electronic device 1200 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

The electrical (conductive) connection member 1271 is a connective member or a connection member, of which one end 1271a may be electrically connected to the conductive portion 533 and the other end 1271b may be connected to the printed circuit board 560. For example, a C-clip may be employed as the electrical connection member 1271.

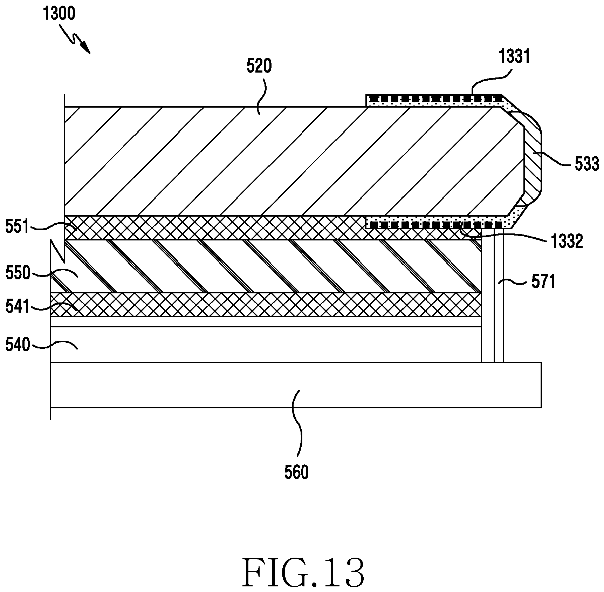

FIG. 13 is a cross-sectional view illustrating an electronic device, which includes various electrode structures capable of measuring biometric information, according to an embodiment of the present disclosure.

Referring to FIG. 13, an electronic device 1300 may be configured to be the same as the electronic device 800 illustrated in FIG. 8, except for the configuration of a first metal mesh 1331 and a second metal mesh 1332.

The electronic device 1300 includes a transparent member 520, a touch panel 550 (e.g., a touch film) coupled to the transparent member 520 by a first transparent adhesive 551 in the second direction of the transparent member 520, a display panel 540 coupled to the touch panel 550 by a second transparent adhesive 541 in the second direction of the touch panel 550, and a printed circuit board 560 spaced apart from the display panel 540 in the second direction. The components listed above may be arranged to overlap each other in the vertical direction. The display module may include the display panel 540 and the touch panel 550. The display may include the display module 540 and 550 and the transparent substrate 520.

The display panel 540 may be disposed such that at least a portion of the display panel 540 is exposed to the outside through the transparent member 520. The first metal mesh 1331 may be disposed to at least partially overlap the display panel 540 (including the touch panel).

The electronic device 1300 may have a second metal mesh 1332 electrically connected to the printed circuit board 560 by the electrical connection device 571.

The electronic device 1300 may measure the user's biometric information (e.g., heartbeat information, body fat information, body water information, or stress information) using at least one first metal mesh 1331 under the control of the control IC (e.g., a sensing circuit) mounted on the printed circuit board 560. The first metal mesh 1331 is an electrode electrically connected to a person's body, while the conductive portion 533 serves as a conductive member that electrically connects the first metal mesh 1331 to the printed circuit board 560 or the control IC.

For example, when the electronic device 1300 is a wearable electronic device worn on the wrist, the first electrode 531 may be touched by and electrically connected to a portion of a person's body (e.g., a finger), and the second electrode 532 may be touched by and electrically connected to a portion of a person's body (e.g., the skin on a wrist).

The electronic device 1300 includes a transparent member 520, a first metal mesh 1331 formed on at least a portion of one face of the transparent member 520, which faces the first direction, and a second metal mesh 1332 formed on at least a portion of the other face of the transparent member 520, which faces the second direction. In FIG. 8, a transparent electrode is used as the first electrode 531, and a transparent/opaque electrode is used as the second electrode 532. According to an embodiment of the present disclosure the first metal mesh 1331 may be employed as the first electrode of the transparent member 520, and the second metal mesh 1332 may be employed as the second electrode. The first and second metal meshes 1331 and 1332 may be arranged to face each other, and may at least partially overlap each other. Each of the first and second metal meshes 1331 and 1332 may be replaced with a transparent polymer conductive material, such as graphene.

When the conductive portion 533 is configured as a metal electrode, the first and second metal meshes 1331 and 1332 and the conductive portion 533 may be formed of the same material (i.e., silver or copper), and the metal line widths of the first and second metal meshes 1331 and 1332 and the conductive portion 533 may be different from each other. The first and second metal meshes 1331 and 1332 and the conductive portion 533 may be formed through a single process, thereby reducing the number of processes.

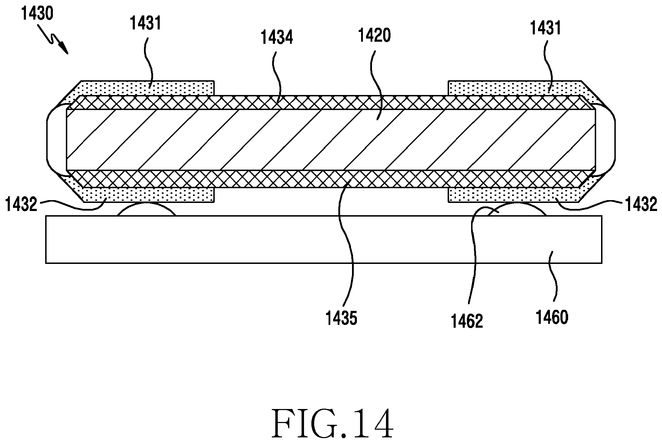

FIG. 14 is a cross-sectional view illustrating an electronic device, which includes various electrode structures capable of measuring biometric information, according to an embodiment of the present disclosure.

Referring to FIG. 14, when the transparent member 1420 is made of PC, acryl, or a synthetic resin material, the electronic device 1430 may have first and second hard coating layers 1434 and 1435 formed on one face, which faces a first direction, and the other face, which faces a second direction, respectively. The first hard coating layer 1434 may be formed entirely on the one face of the transparent member 1420, and the second hard coating layer 1435 may be formed entirely on the other face of the transparent member 1420. At least one first electrode 1431 may be formed on at least a portion of a face of the first hard coating layer 1434, which faces the first direction, and at least one second electrode 1432 may be formed on at least a portion of a face of the second hard coating layer 1435, which faces the second direction. The second hard coat layer 1435 may be omitted if necessary.

The second electrode 1432 may be electrically connected to the printed circuit board 1460 using a connection terminal 1462. The connection terminal 1462 may be an FPCB, a pogo pin, or a metal pin.

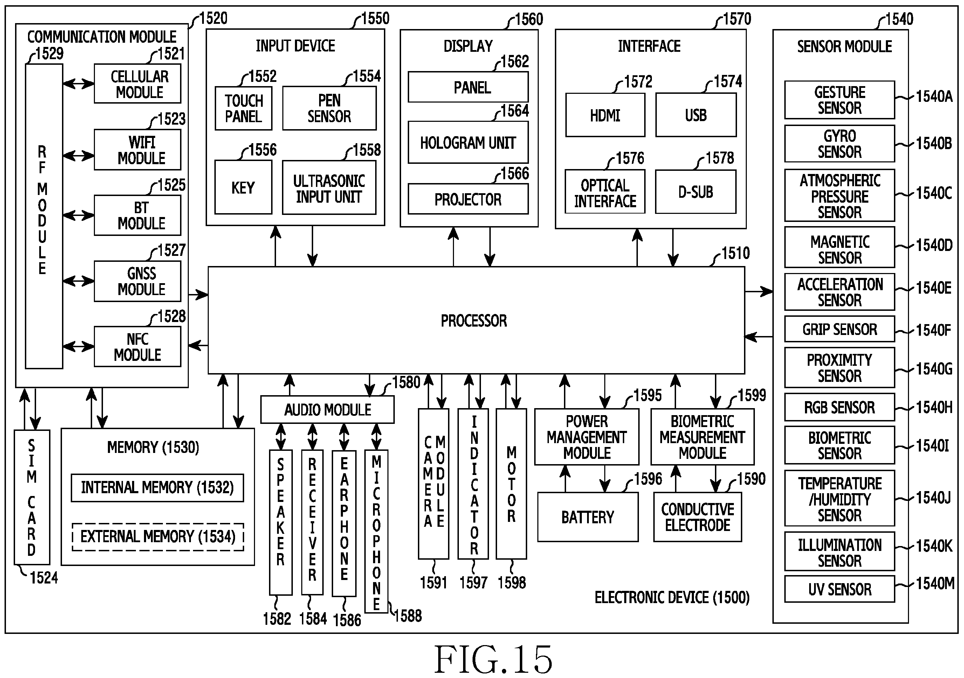

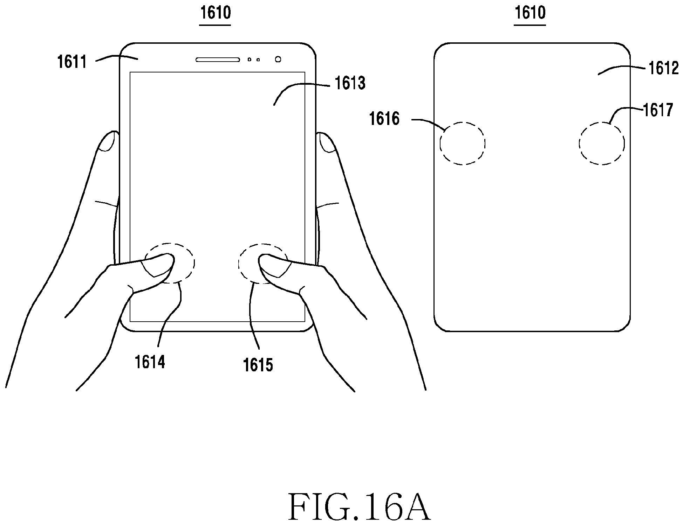

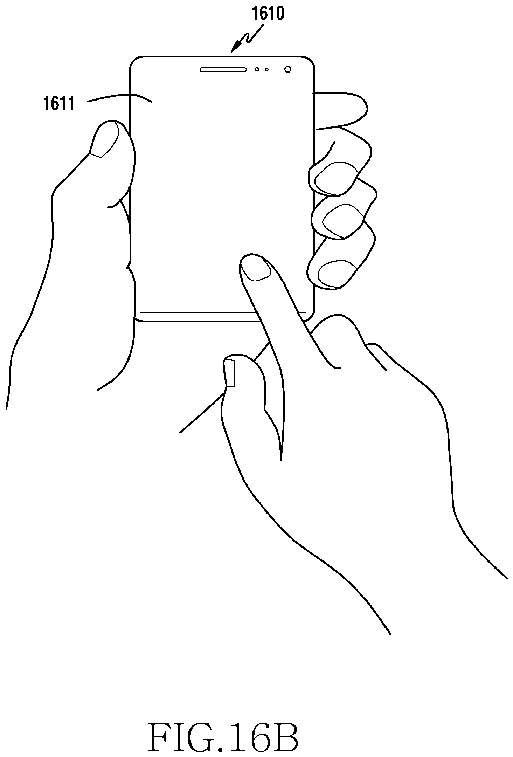

FIG. 15 is a block diagram of an electronic device that provides a biometric measurement function, according to various embodiments of the present disclosure, and FIGS. 16A and 16B are views illustrating an electronic device that provides a biometric measurement function, according to an embodiment of the present disclosure.

Referring to FIG. 15, an electronic device 1500 includes at least one conductive electrode 1590, a biometric measurement module 1599, at least one application processor (AP) 1510, a communication module 1520, a subscriber identification module (SIM) card 1524, a memory 1530, a sensor module 1540, an input device 1550, a display 1560, an interface 1570, an audio module 1580, a camera module 1591, a power management module 1595, a battery 1596, an indicator 1597, and a motor 1598.

The one or more conductive electrodes 1590 may form at least a portion of the outer face of the electronic device 1500. For example, FIGS. 16A and 16B illustrate an electronic device 1610 that provides a biometric measurement function, according to various embodiments of the present disclosure.

Referring to FIG. 16A, the electronic device 1610 is generally in the form of a rectangular plate, and has a first face (or a front face) 1611, which faces a first direction and a second face (or a rear face) 1612, which faces a second direction opposite the first direction. The display 1613 may be disposed in the space between the first face 1611 and the second face 1612, and may be exposed through the first face 1611.

The one or more conductive electrodes 1590 may form at least a portion of the first face 1611. For example, the one or more conductive electrodes 1590 may include one or more front conductive regions (e.g., a first conductive region 1614 and a second conductive region 1615) that form a portion of the first face 1611. One or more front conductive regions 1614 and 1615 may be formed of a light-transmissive material, and may overlap the display 1613. The image-related light output from the display 1613 may be emitted to the outside through the one or more front conductive regions 1614 and 1615.

The one or more conductive electrodes 1590 may form at least a portion of the second face 1612. For example, the one or more conductive electrodes 1590 may also include one or more rear conductive regions (e.g., a third conductive region 1616 and a fourth conductive region 1617) that form a portion of the second face 1612. The one or more rear conductive regions 1616 and 1617 may be formed of a light-transmissive material or a light-blocking material.

One or more front conductive regions or one or more rear conductive regions may have various sizes or shapes, and may also be installed at various locations. The one or more front conductive regions may be designed as a single conductive region extending to cover substantially the entire region of the display 1560.

The biometric measurement module 1599 may detect bioelectricity through one or more conductive electrodes 1590.

For example, referring to FIG. 16A, when two of the user's fingers are touched to the first and second conductive regions 1614 and 1615 of the first face 1611, the user's body may act as a medium that electrically connects the first conductive region 1614 and the second conductive region 1615 to each other.

Referring to FIG. 16B, in a state in which the electronic device 1610 is carried in one hand, when a finger of the other hand is touched to one or more front conductive regions, the user's body may act as a medium that electrically connects one or more rear conductive regions and one or more front conductive regions to each other. Current from the electronic device 1610 may be delivered to the user's body through at least one conductive region, and may be returned to the at least one other conductive area via the user's body. Even if no power is provided to the user's body, the biometric measurement module 1599 may detect a potential difference (e.g., 1mV) caused by, for example, the user's heartbeat, through one or more conductive regions. In a biometric information acquisition operation, the biometric measurement module 1599 may detect an electrical change (e.g., a voltage change, or a current change) caused by the user's body through one or more conductive regions. The biometric measurement module 1599 may deliver the bioelectricity detected through the one or more conductive electrodes 1590 to the AP 1510. According to various embodiments, the biometric measurement module 1599 may be designed to be included in the AP 1510.

The AP 1510 may drive, for example, an operating system or an application program so as to control a plurality of hardware or software components connected to the AP 1510, and may also perform various data processing and arithmetic operations. The AP 1510 may be implemented by a system-on-chip (SoC). The AP 1510 may further include a graphic processing unit (GPU) or an image signal processor. The AP 1510 may include at least some components (e.g., a cellular module 1521) among the components illustrated in FIG. 15. The AP 1510 may load a command or data received from at least one of the other components (e.g., a non-volatile memory) to a volatile memory, thereby processing the command and data, and may store a result in a non-volatile memory.

The AP 1510 may perform at least one operation for acquiring biometric information based at least partially on the execution of an application or the user's input. For example, when a preset user input (e.g., a long touch input with respect to the conductive electrode 1590) is sensed through at least a portion of the input device 1550, the AP 1510 may execute a biometric measurement application to acquire biometric information based on the sensed user input. The AP 1510 may select at least one type of biometric information to be acquired based on at least a setting of an executed biometric measurement application, or the user's input for selecting or requesting biometric information to be measured.

The AP 1510 may identify the type of biometric information to be acquired and select at least one of the conductive electrodes 1590 corresponding to the type of biometric information to be acquired. For example, referring to FIG. 16A, when acquiring first biometric information, the AP 1510 may select at least one of the first conductive region 1614 and the second conductive region 1615 of the first face 1611. In another example, when acquiring second biometric information, the AP 1510 may select at least one of the third conductive region 1616 and the fourth conductive region 1617 of the second face 1612. In another example, when acquiring third biometric information, the AP 1510 may select at least one conductive region of the first face 1611 and at least one conductive region of the second face 1612.

The AP 1510 may acquire biometric information using the at least one selected conductive region and the biometric measurement module 1599. For example, referring to FIG. 16A, when the user's two fingers are touched to the first and second conductive regions 1614 and 1615 of the first face 1611, the user's body may act as a medium that electrically connects the first conductive region 1614 and the second conductive region 1615 to each other. The biometric measurement module 1599 may detect the bioelectricity through the first conductive region 1614 and the second conductive region 1615, may convert the detected bioelectricity into a digital value, and may transmit the digital value to the AP 1510. The AP 1510 may acquire information related to the biometric measurement based on the detected value received from the biometric measurement module 1599. For example, the AP 1510 may analyze the electrical signal (or the detected value) detected through the at least one conductive region using a program related to the biometric measurement, and may acquire the corresponding biometric information.

The AP 1510 may acquire the acquired biometric information, or health information containing the acquired biometric information, and may output the information via the display 1560. The AP 1510 may acquire the acquired biometric information, or health information containing the acquired biometric information, and transmit the information to another electronic device (e.g., an external server that supports healthcare functions) through the communication module 1520.

When one or more front conductive regions 1614 and 1615 are selected based at least partially on the type of biometric information to be acquired, the AP 1510 may variously display one or more regions of the display 1560, which overlaps the selected one or more front conductive regions, using colors and other visual identifiers.

When performing biometric measurement, when at least one conductive region (e.g., the first conductive region 1614 or the second conductive region 1615 of FIG. 16A) disposed on the screen is touched by the user, the AP 1510 may move the content displayed in a region, which includes the touched position to another position, and may display the content in that region.

The AP 1510 may provide information corresponding to physical contact or release by the user to at least one electrode region, and may additionally provide information corresponding to an actuator associated with acoustic or vibrational information associated with a speaker.

The communication module 1520 includes a cellular module 1521, a WiFi module 1523, a Bluetooth module 1525, a global navigation satellite system (GNSS) module 1527, a near field communication (NFC) module 1528, and a radio frequency (RF) module 1529. The cellular module 1521 may provide, for example, a voice call, a video call, a message service, or an internet service through a communication network. The cellular module 1521 may perform discrimination and authentication of the electronic device 1500 within the communication network by using the SIM card 1524. The cellular module 1521 may perform at least some of the functions, which may be provided by the AP 1510. The cellular module 1521 may include a communication processor (CP). At least some (e.g., two or more) of the cellular module 1521, the WiFi module 1523, the Bluetooth module 1525, the GNSS module 1527, and the NFC module 1528 may be incorporated in a single integrated chip (IC) or an IC package.

The RF module 1529 may transmit/receive a communication signal (e.g., an RF signal). The RF module 1529 may include, for example, a transceiver, a power amp module (PAM), a frequency filter, a low-noise amplifier (LNA), or an antenna. At least one of the cellular module 1521, the WiFi module 1523, the Bluetooth module 1525, the GNSS module 1527, and the NFC module 1528 may transmit/receive an RF signal through one or more separate RF modules. The SIM card 1524 may include, for example, an embedded SIM, and may also include unique identification information (e.g., an integrated circuit card identifier (ICCID)) or subscriber information (e.g., an international mobile subscriber identity (IMSI)).

The AP 1510 may transmit acquired biometric information, or health information containing the biometric information, to another electronic device using at least a portion of the communication module 1520.

The memory 1530 may include an internal memory 1532 or an external memory 1534. The internal memory 1532 may include at least one of a volatile memory (e.g., a dynamic random access memory (RAM), a static RAM, or a synchronous dynamic RAM), a non-volatile memory (e.g., an one time programmable read only memory (OTPROM), a programmable read only memory (ROM), an erasable programmable ROM, an electrically erasable programmable ROM, a mask ROM, a flash ROM, or a flash memory), a hard drive, and a solid-state drive (SSD).

The external memory 1534 may further include a flash drive (e.g., a compact flash (CF), a secure digital (SD), a micro secure digital (Micro-SD), a mini secure digital (Mini-SD), an extreme Digital (xD), a multi-media card (MMC), or a memory stick). The external memory 1534 may be functionally or physically connected to the electronic device 1500 through various interfaces.

The memory 1530 may store instructions relating to an operational flow, which is performed by the AP 1510 based on biometric measurement. According to various embodiments of the present disclosure, the memory 1530 may store biometric information acquired at the time of the biometric measurement.

For example, the sensor module 1540 may measure a physical quantity or may sense the operating status of the electronic device 1500, and then convert the measured or sensed information into electric signals. The sensor module 1540 may include at least one of, for example, a gesture sensor 1540A, a gyro sensor 1540B, an atmospheric pressure sensor 1540C, a magnetic sensor 1540D, an acceleration sensor 1540E, a grip sensor 1540F, a proximity sensor 1540G, a color sensor 1540H (e.g., a red, green, blue (RGB) sensor), a biometric sensor 15401, a temperature/humidity sensor 1540J, an illuminance sensor 1540K, and an Ultra-Violet (UV) sensor 1540M. Additionally, or alternatively, the sensor module 1540 may include, for example, an electric nose (E-nose) sensor, an electromyography (EMG) sensor, an electroencephalogram (EEG) sensor, an ECG sensor, an infra-red (IR) sensor, an iris sensor, or a fingerprint sensor. The sensor module 1540 may further include a control circuit for controlling one or more sensors incorporated therein. The electronic device 1500 may further include a processor configured to control the sensor module 1540 as a part of the AP 1510 or separate from the AP 1510 so as to control the sensor module 1540 while the AP 1510 is in a sleep state.

According to various embodiments of the present disclosure, the AP 1510 may determine whether or not the electronic device 1500 is worn or carried by a user based on information from at least a portion of the sensor module 1540. When it is determined that the electronic device 1500 is worn or carried by the user, the AP 1510 may start a series of operations for acquiring biometric information using the one or more conductive electrodes 1590 and the biometric measurement module 1599, based, at least, on biometric information to be acquired.

According to an embodiment of the present disclosure, the biometric sensor 15401 may replace the biometric measurement module 1599, so that the biometric measurement module 1599 may not be included in the electronic device 1500. In this case, the one or more conductive electrodes 1590 may be connected to the biometric sensor 15401.

The input device 1550 may include, for example, a touch panel 1552, a (digital) pen sensor 1554, a key 1556, or an ultrasonic input device 1558. As the touch panel 1552 may use, at least one of, for example, a capacitive type touch panel, a resistive-type touch panel, an infrared-type touch panel, and an ultrasonic type panel. Also, the touch panel 1552 may further include a control circuit and a tactile layer so as to provide a tactile reaction to the user. The (digital) pen sensor 1554 may be, for example, a portion of the touch panel, or may include a separate recognition sheet. The key 1556 may include, for example, a physical button, an optical key, or a keypad. The ultrasonic input device 1558 may sense, through a microphone 1588, ultrasonic waves generated by an input tool so as to enable confirmation of data corresponding to the sensed ultrasonic waves.

The display 1560 includes a panel 1562, a hologram device 1564, a projector 1566, or a control circuit for controlling these components. The panel 1562 may be implemented to be, for example, flexible, transparent, or wearable. The panel 1562 may include the touch panel 1552 and one or more modules. According to one embodiment, the panel 1562 may include a pressure sensor (or a force sensor) that is capable of measuring the intensity of pressure of a user's touch. The pressure sensor may be integrally implemented with the touch panel 1552, or may be implemented as one or more sensors separate from the touch panel 1552. The hologram device 1564 may show a stereoscopic image in the air using interference of light. The projector 1566 may project light onto a screen so as to display an image. The screen may be located, for example, inside or outside the electronic device 1500. The interface 1570 may include, for example, a high definition multimedia interface (HDMI) 1572, a universal serial bus (USB) 1574, an optical interface 1576, or a D-subminiature (D-sub) interface 1578. Additionally, or alternatively, the interface 1570 may include, for example, a mobile high-definition link (MHL) interface, an SD card/MMC interface, or an Infrared Data Association (IrDA) standard interface.

The audio module 1580 may bi-directionally convert sound and electric signals. The audio module 1580 may process sound information input or output through, for example, a speaker 1582, a receiver 1584, an earphone 1586, or the microphone 1588. According to various embodiments of the present disclosure, at the time of biometric measurement, the audio module 1580 may output various sounds associated with the biometric measurement function through the speaker 1582 under the control of the AP 1510.

The power management module 1595 may manage, for example, the electric power of the electronic device 1500. The power management module 1595 may include a power management IC (PMIC), a charger IC, or a battery gauge. The PMIC may be configured as a wired or wireless charging type. The wireless charging type may include, for example, a magnetic-resonance type, a magnetic-induction type, or an electromagnetic-wave type, and may further include an additional circuit for wireless charging (e.g., a coil loop, a resonance circuit, or a rectifier). The battery gauge may measure the remaining charge of the battery 1596, and a voltage, a current, or a temperature at the time of the charge. The battery 1596 may include, for example, a rechargeable battery or a solar battery.

According to various embodiments of the present disclosure, the biometric measurements may be divided into a first type of biometric measurement and a second type of biometric measurement. The first type of biometric measurement may be a mode of applying power to at least one conductive electrode 1590 and detecting bioelectricity through the at least one conductive electrode 1590 so as to acquire corresponding biometric information. The second type of biometric measurement may be a mode of detecting bioelectricity through the at least one conductive electrode 1590 to acquire corresponding biometric information without applying power to the at least one conductive electrode 1590. When performing the first biometric measurement, the AP 1510 may adjust the power management module 1595 such that power is provided to the at least one conductive electrode 1590. When performing a second biometric measurement, the AP 1510 may adjust the power management module 1595 such that power is not provided to the at least one conductive electrode 1590.

The indicator 1597 may indicate a specific status (e.g., a booting status, a message status, or a charged status) of the electronic device 1500 or of a part thereof (e.g., AP 1510). According to various embodiments of the present disclosure, in the biometric measurement modes, the indicator 1597 may display a biometric measurement-related event under the control of the AP 1510.

The motor 1598 may convert an electric signal into a mechanical vibration, and may generate, for example, a vibration or a haptic effect. According to various embodiments of the present disclosure, at the time of a biometric measurement, the motor 1598 may generate a vibration for a biometric measurement-related event under the control of the AP 1510.

The camera module 1591 is a device that is capable of capturing an image, for example, a still image and a video image, and according to one embodiment, the camera module 1591 may include at least one image sensor (e.g., a front sensor or a rear sensor), a lens, an image signal processor (ISP), or a flash (e.g., an LED or xenon lamp).

The electronic device 1500 may include, for example, a mobile TV support device (e.g., a GPU) that is capable of processing media data according to a standard of, for example, digital multimedia broadcasting (DMB), digital video broadcasting (DVB), or MediaFlo™.

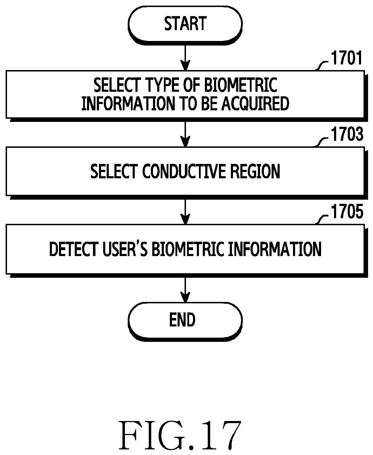

FIG. 17 is a flowchart of an operation for a biometric measurement function in an electronic device, according to an embodiment of the present disclosure.

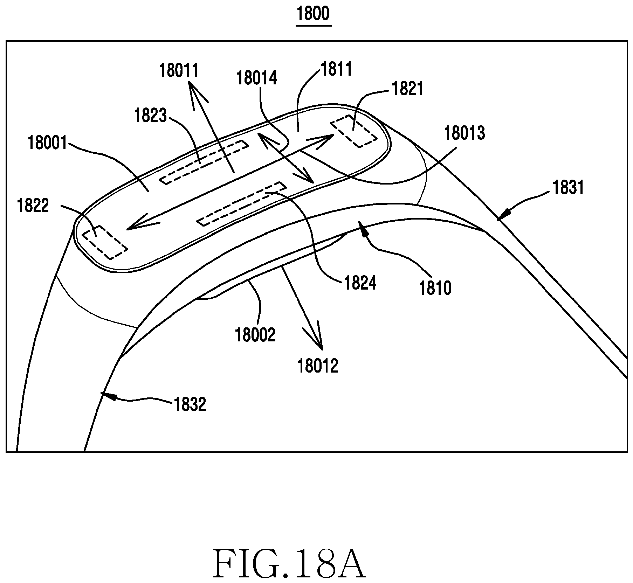

FIG. 18A illustrates an electronic device, which performs the operation of FIG. 17, according to an embodiment of the present disclosure.

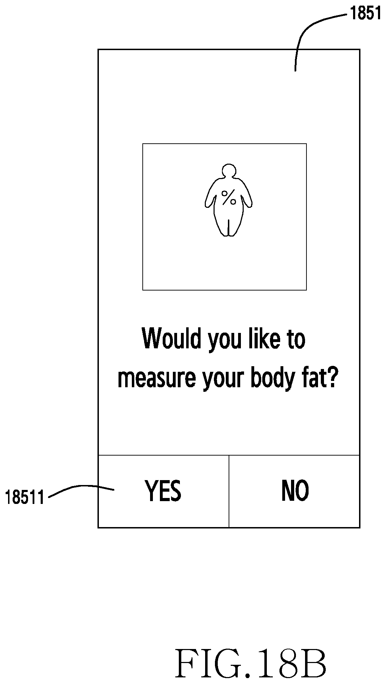

FIGS. 18B to 18D are views for describing the operation of FIG. 17, according to an embodiment of the present disclosure.

Referring to FIG. 17, in step 1701, the processor (e.g., the AP 1510 of FIG. 15) selects the type of biometric information to be acquired based at least partially on the execution of an application or user input. For example, the electronic device 1500 may execute a biometric measurement application when a corresponding icon is selected (e.g., touched) or when receipt of a preset user input (e.g., a set gesture input) is confirmed. Depending on the executed biometric measurement application, the AP 1510 may display a screen that provides a list of various biometric measurement functions (e.g., a list of types of measurable biometric information) (hereinafter, referred to as a "biometric measurement list"). When it is detected that at least one biometric measurement entry (e.g., a type of biometric information to be measured) in the biometric measurement list is selected by at least one of a user input and a biometric measurement application setting, the AP 1510 may perform a series of operations related to biometric measurement (e.g., acquisition of biometric information) corresponding to at least one selected biometric measurement entry.

In step 1703, the AP 1510 selects at least one conductive region formed on the outer face of the electronic device 1500 based at least on the selected biometric measurement entry.

For example, FIG. 18A illustrates an electronic device 1800 that provides biometric measurement function. The electronic device 1800 may be a watch that includes a body 1810, a display 1811 and extensions 1831 and 1832 (e.g., bands or straps) connected to both sides of the body 1810. The body 1810 includes a first face 18001 facing a first direction 18011 and a second face 18002 facing a second direction 18012 that is opposite the first direction 18011. The display 1811 may be disposed between the first face 18001 and the second face 18002, and maybe exposed through the first face 18001.

When the electronic device 1800 is worn on the user's wrist, the second face 18002 may be in contact with the user's wrist. The electronic device 1800 may include at least one light-transmissive and conductive region (or a conductive electrode) that forms at least a portion of the first face 18001. The at least one light-transmissive and conductive region may overlap the display 1811. At least one light-transmissive and conductive region includes a first conductive region 1821 and a second conductive region 1822, which are arranged in a direction 18013 between the extensions 1831 and 1832, and a third conductive region 1823 and a fourth conductive region 1824, which are arranged in a direction 18014 orthogonal to the direction 18013.

The AP 1510 may select at least one of a plurality of conductive regions 1821, 1822, 1823, and 1824 of the first face 18001 to correspond to the selected biometric measurement entry. For example, when any one biometric measurement entry is selected, the AP 1510 may select the first conductive region 1821 and the second conductive region 1822 of the first face 18001. When the user's two fingers are touched to the first and second conductive regions 1821 and 1822 of the first face 18001, the user's body may act as a medium that electrically connects the first conductive region 1821 and the second conductive region 1822 to each other.

The electronic device 1800 may include at least one conductive region that forms at least a portion of the second face 18002. The AP 1510 may select at least one conductive region of the second face 18002 according to the selected biometric measurement entry. For example, the AP 1510 may select two conductive regions of the second face 18002 corresponding to the selected biometric measurement entry (e.g., corresponding to the type of biometric information to be acquired). When the electronic device 1800 is worn on the user's wrist, the two selected conductive reasons are in contact with the user's wrist, and the user's body may act as a medium that electrically connects the two conductive regions to each other.

The AP 1510 may select at least one conductive region of the first side 18001 and at least one conductive region of the second side 18002 according to the selected biometric measurement entry. When the electronic device 1800 is worn on the user's wrist, at least one conductive region disposed on the second face 18002 may be in contact with the user's wrist. In the state in which the electronic device 1800 is worn on the user's wrist, when at least one light-transmissive and conductive region of the first face 18001 is touched by the user's finger with the electronic device 1800, the user's body may act as a medium that electrically connects the at least one conductive region of the second face 18002 and the at least one light-transmissive region of the first face 18001 to each other.

When a BIA biometric measurement entry is selected, the AP 1510 may select four conductive regions (or conductive electrodes) among the plurality of conductive regions formed on the outer faces (e.g., the first face 18001 and the second face 18002) of the electronic device 1800. For example, in the BIA biometric measurement entry, the AP 1510 may select two conductive regions of the first face 18001 and two conductive regions of the second face 18002.

When an ECG biometric measurement entry is selected, the AP 1510 may select three conductive regions among the plurality of conductive regions formed on the outer faces of electronic device 1800. For example, when the ECG biometric measurement entry is selected, the AP 1510 may select one conductive region of the first face 18001 and one conductive region of the second face 18002.

When a GSR biometric measurement entry is selected, the AP 1510 may select two conductive regions among the plurality of conductive regions formed on the outer faces of electronic device 1800. For example, when the GSR biometric measurement entry is selected, the AP 1510 may select two conductive regions of the second face 18002.

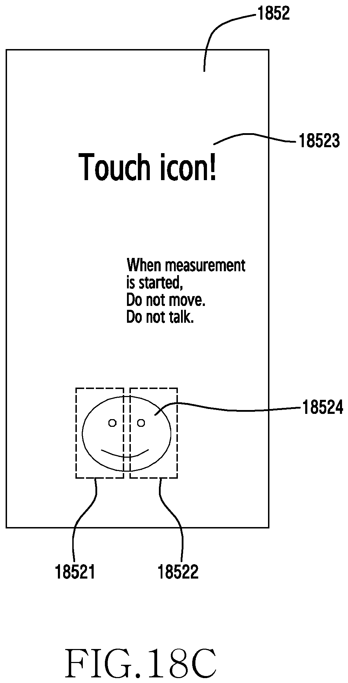

Referring to FIG. 18B, the AP 1510 provides a first screen 1851 capable of receiving the user's selection for any one biometric measurement entry (e.g., a BIA biometric measurement). When a region 18511 for selecting a biometric measurement entry in the first screen 1851 is touched, the AP 1510 may control switching to a second screen 1852 illustrated in FIG. 18C. According to various embodiments of the present disclosure, one or more conductive regions for the selected biometric measurement entry may overlap the display (e.g., the display 1811 of Figure 18A). For example, referring to FIG. 18C, the AP 1510 may select two conductive regions 18521 and 18522, which overlap the display, based on the selected biometric measurement entry.

Referring to Figure 18C, the AP 1510 may display a screen region 18524, which at least partially overlaps both conductive regions 18521 and 18522. For example, an image, such as an icon may be displayed in the screen region 18524. The second screen 1852 may provide guidance information 18523 for guiding the user to touch the displayed screen region 18524. The displayed screen region 18524 is capable of enabling the user to recognize the positions of the conductive regions 18521 and 18522 (e.g., the positions of the electrodes) corresponding to the selected biometric measurement entry. For example, when one finger of the user is moved close to the screen region 18524, which is displayed as an image for biometric measurement, both of the conductive regions 18521 and 18522 may be touched by the user's finger.

In step 1705, the AP 1510 acquires user biometric information through the at least one selected conductive region. According to various embodiments of the present disclosure, the electronic device 1800 may include a biometric measurement circuit (e.g., a biosensor or a bioprocessor) (e.g., the biometric measurement module 1590 of FIG. 15) electrically connected to the at least one selected conductive region. The biometric measurement circuit may include an analog-to-digital converter (ADC). The biometric measurement circuit may detect bioelectricity through the at least one selected conductive region, and the ADC may generate (e.g., quantize) a corresponding digital value (or an ADC value). The AP 1510 may acquire biometric information related to the biometric measurement based on the detected value through at least one conductive region. For example, the AP 1510 may analyze the electrical signal (or the detected value) detected through the at least one conductive region using a program related to the biometric measurement, and acquire the corresponding biometric information.

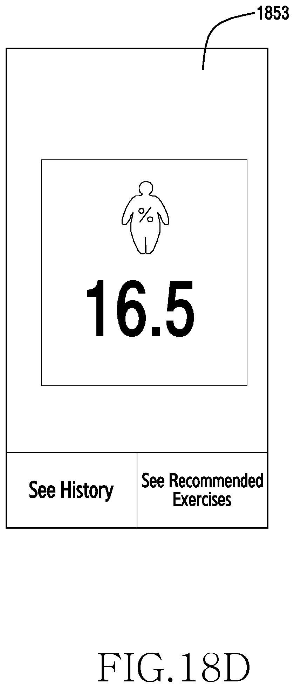

Referring to FIG. 18D, the AP 1510 may output biometric information acquired through the biometric measurement or information including the biometric information (e.g., acquired biometric information and previously acquired biometric information) through the display. The AP 1510 may switch from the second screen 1852 of FIG. 18C to the third screen 1853 of FIG. 18D and output the biometric information through the third screen 1853. The AP 1510 may transmit biometric information acquired through the biometric measurement to other electronic devices. For example, the AP 1510 may transmit the acquired biometric information to a server that supports a health care function.

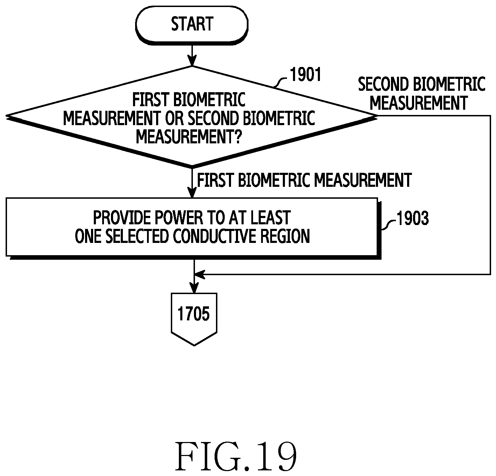

FIG. 19 is a flowchart of an operation related to power control in an electronic device that provides a biometric measurement function, according to an embodiment of the present disclosure.

According to various embodiments of the present disclosure, the operation of FIG. 19 may be implemented between steps 1703 and 1705 of FIG. 17.

Referring to FIG. 19, in step 1901, the processor (e.g., the AP 1510 of FIG. 15) determines whether a selected biometric measurement entry (e.g., the type of biometric information to be acquired or measured) is a first biometric measurement or a second biometric measurement. The first biometric measurement is performed by applying power (e.g., current or voltage) to at least one conductive region and detecting a change in current or voltage with respect to bioelectricity (or biometric information) over time through the at least one conductive region. For example, the first biometric measurement may include a BIA biometric measurement, a GSR biometric measurement, or a skin temperature biometric measurement. The second biometric measurement may be a mode of detecting a change in voltage with respect to bioelectricity (or biometric information) over time through at least one conductive region without applying power to the at least one conductive region. For example, the second biometric measurement may include an ECG biometric measurement or an EMG biometric measurement.

When the selected biometric measurement entry (e.g., the type of acquired or measured biometric information) corresponds to the first biometric measurement, the AP 1510 performs step 1903. In step 1903, the AP 1510 adjusts a power management device (e.g., a PMIC) (e.g., a power management module 1595 of FIG. 15) such that power is provided to at least one selected conductive region.

The AP 1510 may perform step 1705 of FIG. 17 after providing power to at least one conductive region selected in step 1903. For example, at the time of the biometric measurement corresponding to the first biometric measurement, the current from the electronic device 1500 may be delivered to the user's body through one conductive region, and may be returned to another conductive region via the user's body. At the time of the biometric measurement corresponding to the first biometric measurement, the AP 1510 may detect an electrical change (e.g., a voltage change, or a current change) caused by the user's body, and may acquire information about the first biometric measurement therefrom.

At the time of the biometric measurement corresponding to the second biometric measurement, the AP 1510 may perform step 1705 in FIG. 17, and adjust the power management device such that power (e.g., current or voltage) is not provided to the at least one selected conductive region. The AP 1510 may detect a change in voltage with respect to the bioelectricity (or biometric information) through at least one conductive region in the state in which no power is applied to the at least one selected conductive region. For example, at the time of the ECG biometric measurement, a potential difference (e.g., 1 mV) caused by the user's heartbeat may be detected through at least one conductive region.

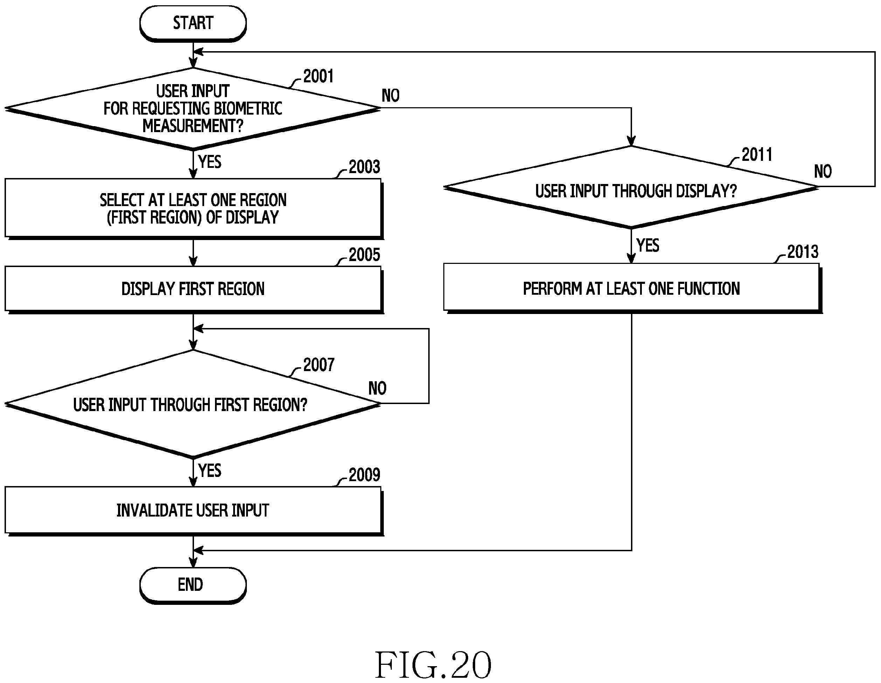

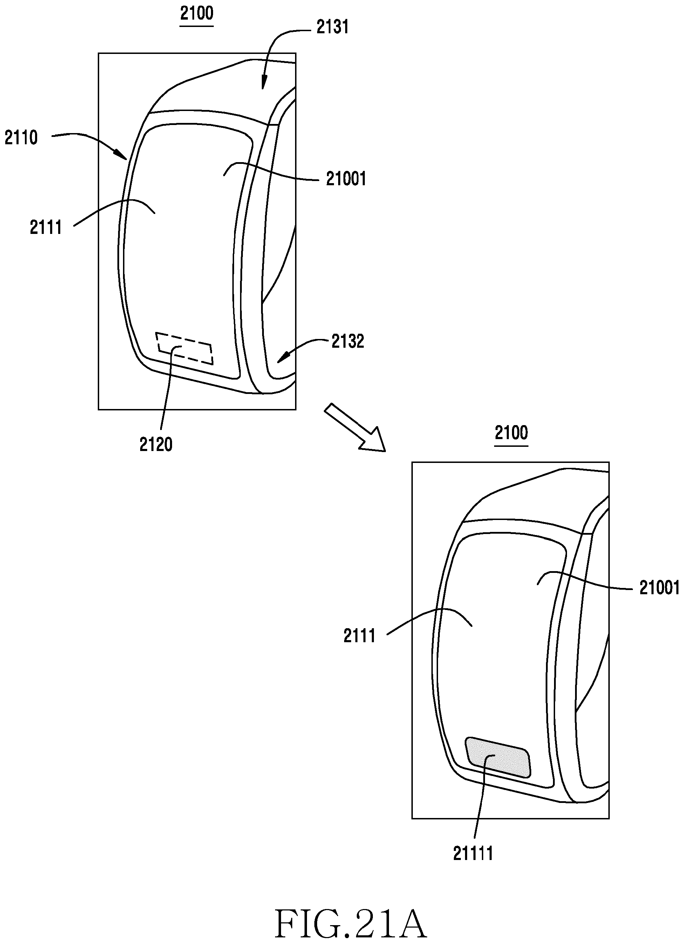

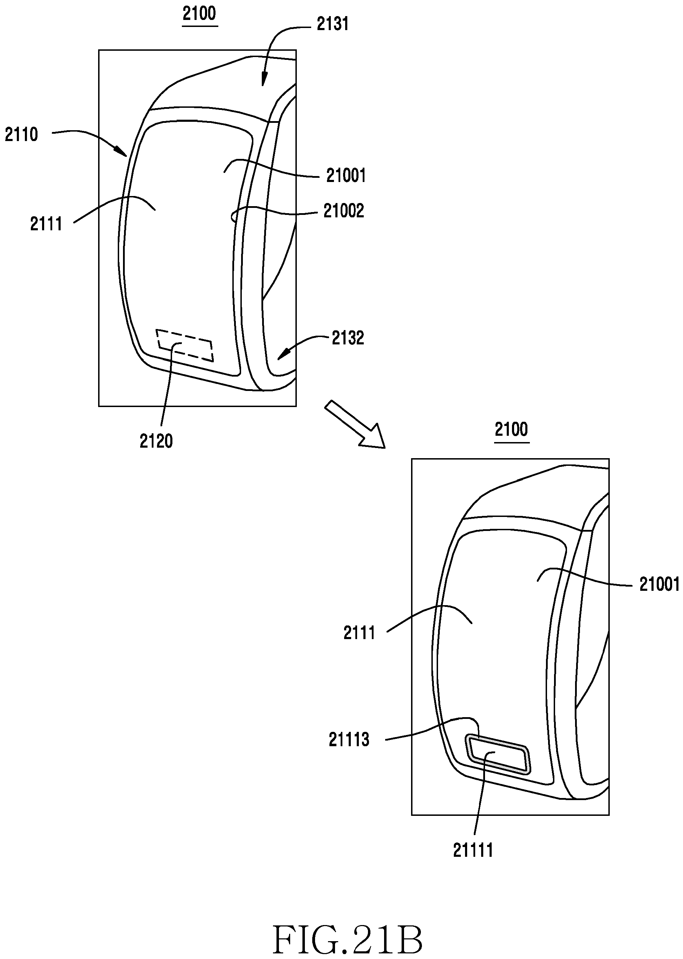

FIG. 20 is a flowchart of an operation related to input or output through a display in an electronic device that provides a biometric measurement function, according to an embodiment of the present disclosure. FIGS. 21A and 21B are exemplary views for describing the operation of FIG. 20, according to various embodiments of the present disclosure.

Referring to FIG. 20, in step 2001, whether or not the user input requesting biometric measurement is received is determined, and when it is determined that the user input requesting biometric measurement is received, the processor (e.g., the AP 1510 of FIG. 15) may perform step 2003. In various embodiments of the present disclosure, an operation of receiving a user input for selecting a biometric measurement entry (the type of biometric information to be measured) may precede step 2003. When it is determined that the user input requesting biometric measurement is received, at least one predesignated biometric measurement entry may be automatically selected. For example, when it is determined that the user input requesting biometric measurement is received, the AP 1510 may automatically select a biometric measurement entry (e.g., a BIA biometric measurement) based on the setting. The biometric measurement requested by the user input may be the first biometric measurement or the second biometric measurement described above with reference to FIG. 19.

In step 2003, the AP 1510 may select at least one region of the display based at least on the biometric measurement entry (e.g., the type of biometric information to be acquired). The biometric measurement entry may be selected by the user, or selected in a predetermined order based on the setting of the biometric measurement application.

Referring to FIG. 21A, the AP 1510 selects at least one region (e.g., the first region 2120) of the display 2111 based on the biometric measurement entry. Although the display 2111 is illustrated as including only the first region 2120, the display 2111 according to various embodiments may further include additional regions (e.g. regions 1821, 1822, 1823, and 1824 illustrated in FIG. 18A) other than the first region 2120. At least a portion of the first region 2120 may include a light-transmissive region or a conductive region, and may be electrically connected to the AP 1510.

In step 2005, the AP 1510 displays the selected first area. The electronic device 2100 may be a watch including a body 2110, a display 2111 and extensions 2131 and 2132 connected to both sides of the body 2110. The display 2111 may be designed to extend along at least a portion of the first face 21001. The first face 21001 may be generally planar or curved. At the time of the biometric measurement, the AP 1510 may display the first region 2120 to be visually distinguished from other regions of the display 2111 (e.g., by yellow, red, or black), as illustrated by 21111 in FIG. 21A.

At the time of the biometric measurement, the AP 1510 may display a portion of the first region 2120 (e.g., the periphery region surrounding the first region), which at least partially overlaps at least one light-transmissive and conductive region, to be visually distinguished from other regions of the display 2111.

For example, referring to Figure 21B, at the time of the biometric measurement, the AP 1510 may display an edge 21113 surrounding the first region 2120 with a color that is visually distinguished from another region or the first region 2120 of the display 2111. At the time of the biometric measurement, the user may recognize the position of the conductive region (e.g., the first region 2120) to be proximate or necessary to be touched for the biometric measurement.

In addition, the first region 2120 can be displayed by various methods not illustrated. For example, the AP 1510 may display the first region 2120 or the edge 21113 in a blinking/flashing manner. The AP 1510 may perform control such that the first region 2120 and the edge 21113 are displayed to be visually distinguished from each other, and such that the first region 2120 or the edge 21113 is displayed to be visually distinguished from other regions of the display 2111.

At least one light-transmissive and conductive region may be designed to have a color. For example, the at least one light-transmissive region may have a material having light transmittance and a color. When the at least one light-transmissive region is designed to have a color, step 2005 may be omitted.

In step 2007, the AP 1510 determines whether user input is sensed through the first region 2120. Referring to FIG. 21A or 21B, the display 2111 may include a touch screen panel, and may receive user input made using, for example, an electronic pen or a portion of a user's body, such as a touch input, a gesture input, a proximity input, or a hovering input. When a finger of the user is moved closer to the first region 2120 for biometric measurement, the user's finger may touch the light-transmissive and conductive region 2120 covering the first region 2120, and the AP 1510 may sense the user input through the first region 2120. When the user's finger touches the light-transmissive and conductive region 2120, the AP 1510 may detect the bioelectricity through the light-transmissive and conductive region 2120 and may acquire information (e.g., biometric information) related to the biometric measurement based on the detected value (e.g., step 1705 in FIG. 17).

When user input is sensed through the first region 2120, the AP 1510 may perform step 2009. In step 2009, the AP 1510 invalidates the user input sensed through the first region 2120. The AP 1510 may perform a function related to the biometric measurement based on the user input sensed through the first region 2120.

In the case in which biometric measurement is performed through at least one biometric information measurement region (e.g., the first region 2120) that is selected to correspond to the biometric measurement entry, the AP 1510 may invalidate the user input (e.g., a touch input) sensed through the display 2111, the first region 2120, or the periphery of the first region (e.g., the edge 21113 in FIG. 21B). When biometric measurement is performed, the AP 1510 may detect bioelectricity through the first region 2120 while invalidating the touch input sensed through the first region 2120 or the periphery of the first region (e.g., the edge 21113 in FIG. 21B).

While the biometric measurement is performed, the AP 1510 may deactivate the region of the touch panel, which corresponds to the first region 2120 or the periphery of the first region (e.g., the edge 21113).

When a touch input is sensed through the first display 2111, the AP 1510 may release the display of the first display 2111 or switch to another color.

The AP 1510 may determine the duration time of the touch input sensed through the first region 2120, and may use the same for biometric measurement. For example, the AP 1510 may display guidance information for guiding the user through the display 2111 such that the duration time of the touch input through the first region 2120 may be equal to longer than a reference time (e.g., 10 seconds). For example, the AP 1510 may display the duration time of the touch input sensed through the first region 2120 through the display 2111, and when the duration time is equal to or longer than a defined time, the AP 1510 may display, through the display 2111, guidance information for guiding the user to remove the finger of the user from the first region 2120.

The AP 1510 may detect the touch area (or the touch contact area) of the touch input sensed through the first region 2120, and may use the same for a biometric measurement. The touch area may be defined as a set of a plurality of touch points. When the touch area is smaller than a threshold value, the AP 1510 may display the guidance information through the display 2111 so as to guide the user to bring the user's finger into closer contact therewith, thereby increasing the touch area.

The AP 1510 may perform various other functions based on the touch input sensed through the first region 2120.

In step 2011, the AP 1510 determines whether user input occurs through the display. When user input is determined not to occur through the display in step 2011, the AP 1510 performs step 2001 again.

When user input occurs through the display in step 2011, the AP 1510 performs step 2013. In step 2013, the AP 1510 performs at least one function according to the user input. For example, when an icon displayed on the screen is selected via the user input, a corresponding application may be executed.

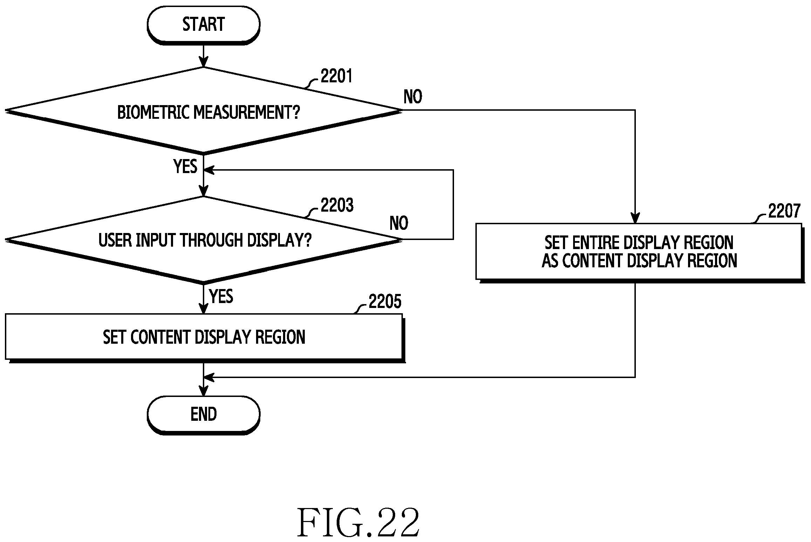

FIG. 22 is a flowchart of an operation related to input or output through a display in an electronic device that provides a biometric measurement function, according to an embodiment of the present disclosure.

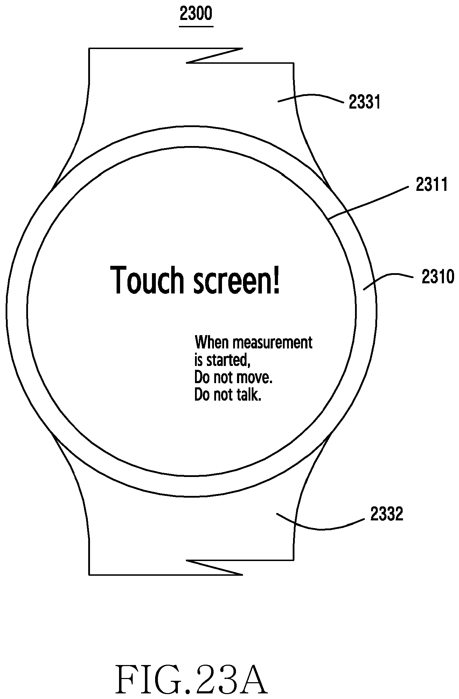

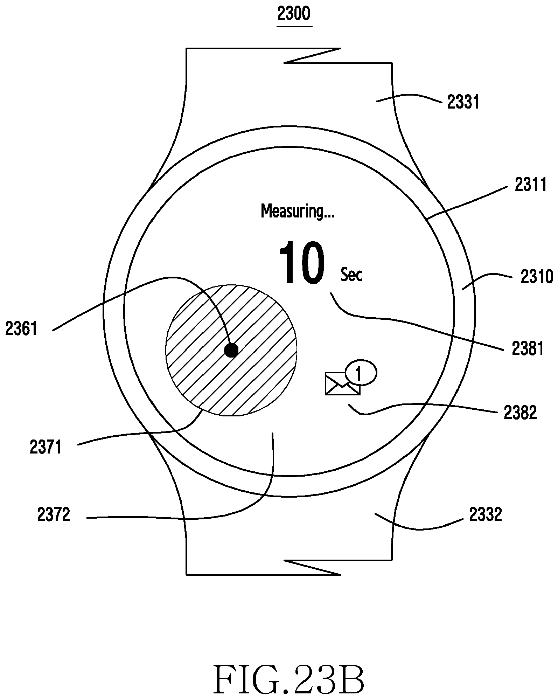

FIGS. 23A and 23B are views illustrating an electronic device that provides a biometric measurement function, according to various embodiments of the present disclosure.

Referring to FIGS. 23A and 23B, the electronic device 2300 may be a watch including a body 2310, a display 2311, and extensions 2331 and 2332 connected to both sides of the body 2310. The electronic device 2300 may include a light-transmissive and conductive region covering at least a partial region of the display 2311.

The display 2311 may have a substantially circular shape, and the light-transmissive and conductive region may be designed in a size extending to cover the entire region of the display 2311.

Referring to FIG. 22, when performing biometric measurement at step 2201, the processor (e.g., the AP 1510 of FIG. 15) performs step 2203. In step 2201, the AP 1510 determines whether to perform biometric measurement based at least on the acknowledgment of receipt of user input requesting the biometric measurement. The biometric measurement requested by the user input may be the first biometric measurement or the second biometric measurement described above with reference to FIG. 19.

In step 2203, the AP 1510 determines whether the user input (e.g., proximity or touch) occurs through the display 2311 (e.g., a touch screen). Referring to FIG. 23A, at the time of the biometric measurement, the AP 1510 may display, through the display 2311, guidance information for guiding the user to touch the screen.

When user input (e.g., touch or proximity) is received through the display 2311 in step 2203, the AP 1510 may perform step 2205.

In step 2205, the AP 1510 sets a content display region based at least on the user input (e.g., touch). Referring to FIG. 23B, the AP 1510 may divide the display region of the display 2311 into a first region 2371 having a predetermined radius from a touch point 2361 and a second region 2372 outside the first region 2371, and may set the second region 2372 as the content display region. The first region 2371 and the second region 2372 may be distinguished as a region in which biometric information may be acquired and a region in which contents may be displayed, respectively. However, the second region 2372 may also be designed to acquire biometric information.

For example, when the user touches the second region 2372 while acquiring the biometric information through the first region 2371 and displaying the content in the second region 2372, a region including the touch point in the second region 2372 becomes the region from which the biometric information can be obtained and the remaining region of the second region 2372 or the first region 2371 may be switched to a region for displaying the content. The AP 1510 may set a content display region based at least on the touch area (or the touch contact region). The content display region (e.g., the second region) may be a region that does not substantially overlap the touch contact region. The AP 1510 may adjust the content to be displayed through the set content display region. The content (e.g., an icon) displayed through the first region 2371 may be moved to and displayed in the second region 2372 when a touch to the first region 2371 is sensed.

The AP 1510 may display the first region 2371 to be visually distinguished from the second region 2372 (e.g., to be distinguished by colors such as yellow, red, and black).

The AP 1510 may display, through the second area 2372, a content 2381 (e.g., elapsed time of biometric measurement and a time remaining until completion of the biometric measurement) related to the biometric measurement. The AP 1510 may display various types of contents (e.g., SMS messages, SNS messages, or chat messages) generated in an electronic device 2300 or received from the outside, through the second region 2372 (e.g, 2382). For example, at the time of biometric measurement, the contents displayed in the second region 2372 may be visible to the user without being obscured by a finger of the user.

While a user is touching the display 2311, the AP 1510 may detect bioelectricity through the light-transmissive and conductive region (e.g., the first region 2371), and may acquire information related to an activated biometric measurement therefrom (e.g., step 1705 in FIG. 17).

The AP 1510 may perform step 2205 when the duration time of the touch on display 2311 is equal to or longer than a reference time. When the duration time of the touch on the display 2311 is equal to or longer than a reference time, the AP 1510 may start a series of operations for detecting and analyzing bioelectricity through the light-transmissive and conductive region (e.g., the first region 2371).

When a touch-contact region on the display 2311 is equal to or larger than a reference region, the AP 1510 may perform step 2205. When the touch contact region on the display 2311 is equal to or larger than the reference region, the AP 1510 may start a series of operations for detecting and analyzing bioelectricity through the light-transmissive and conductive region (e.g., the first region 2371).

In step 2207, the AP 1510 sets the entire region of the display as a content display region.

The embodiments of the present disclosure may also be implemented as a computer program executed in a computer which executes the program using a non-transitory computer-readable medium. A data structure for executing the program may be recorded on the computer-readable medium including storage media such as magnetic storage media (e.g., ROM, floppy disks, or hard disks) and optical recording media (e.g., CD-ROM or DVD).

While the present disclosure has been shown and described with reference to certain embodiments, it will be understood by those skilled in the art that various changes in form and details may be made therein without departing from the scope of the present disclosure, which is defined, not by the detailed description and embodiments, but by the appended claims.

## Claims

1. An electronic device (100, 200, 300, 400, 600, 1500) comprising:
a housing (111, 210, 310, 410, 610);
a display panel (112, 240, 340) at least partially accommodated in the housing;
a first electrode (431, 631) formed over at least a portion of the display panel to be visually transparent and to be exposed to the outside of the electronic device;
a second electrode (432, 632) formed in at least a portion of one face of the housing below the display panel to be exposed to the outside of the electronic device;
a transparent member (341, 420, 620) formed between the display panel (112, 240, 340) and the first electrode (431, 631);
a conductive layer (433, 633) formed on at least a portion of a side face of the transparent member (341, 420, 620), wherein an end portion of the conductive layer (433, 633) is electrically connected to the first electrode (431, 631); and
a processor (1510) configured to display, through the display panel (112, 240, 340), guidance information for indicating an area of the display panel which overlaps the first electrode,
wherein the first electrode (431, 631) is formed on at least a portion of a face of the transparent member (341, 420, 620), which faces the outside,
wherein at least a portion of a boundary region between the face and the side face of the transparent member forms a chamfered region, and the first electrode (431, 631) and the conductive layer (433, 633) at least partially overlap each other in the chamfered region, and
wherein, when the first electrode (431, 631) is in contact with a first body portion of a user of the electronic device and the second electrode (432,632) is in contact with a second body portion of the user, the first electrode and the second electrode are electrically connected to each other.

2. The electronic device (100, 200, 300, 400, 600, 1500) of claim 1, further comprising a conductive member (533) electrically connected to a remaining end portion of the conductive layer and to a sensing circuit.

3. The electronic device (100, 200, 300, 400, 600, 1500) of claim 1, wherein the processor (1510) is further configured to display at least a portion of the guidance information through a remaining region of the display panel, which does not overlap the first electrode.

4. The electronic device (100, 200, 300, 400, 600, 1500) of claim 3, wherein the processor is further configured to display the guidance information using graphic information.

5. The electronic device (100, 200, 300, 400, 600, 1500) of claim 1 further comprising a transparent member (341, 420, 620) coupled to the housing to cover the display panel (112, 240, 340), thereby forming one face of the electronic device; and
a printed circuit board (321, 560) accommodated in the housing (111, 210, 310, 410, 610), and having a control circuit disposed therein electrically connected to the first electrode (431, 631) and the second electrode (432, 632),
wherein the first electrode (431, 631) is substantially transparent and is formed on a face of the transparent member (341, 420, 620), which faces the outside of the electronic device and the second electrode (432, 632) is formed on a face of the housing (111, 210, 310, 410, 610), which is opposite the face of the transparent member (341, 420, 620); and
wherein the control circuit is configured to measure a user's biometric information using an electrical signal acquired through the first electrode (431, 631) or the second electrode (432, 632).

6. The electronic device (100, 200, 300, 400, 600, 1500) of claim 5, further comprising a conductive portion formed on a side face of the transparent member (341, 420, 620) to be in contact with the first electrode (431, 631).

7. The electronic device (100, 200, 300, 400, 600, 1500) of claim 6, wherein at least a portion of the conductive portion is visually opaque.

8. The electronic device (100, 200, 300, 400, 600, 1500) of claim 6, further comprising a conductive connection member, one end portion of which is electrically connected to the conductive portion and a remaining end portion of which is electrically connected to the printed circuit board (321, 560).

9. The electronic device (100, 200, 300, 400, 600, 1500) of claim 5, wherein the control circuit is further configured to change a color of the first electrode (431, 631) in connection with a measurement of the biometric information.

## Patentansprüche

1. Elektronische Vorrichtung (100, 200, 300, 400, 600, 1500), welche Folgendes umfasst:
ein Gehäuse (111, 210, 310, 410, 610);
ein Anzeigefeld (112, 240, 340), das zumindest teilweise in dem Gehäuse untergebracht ist;
eine erste Elektrode (431, 631), die über mindestens einem Teil des Anzeigefeldes gebildet ist, um visuell transparent zu sein, und um der Außenseite der elektronischen Vorrichtung ausgesetzt zu sein;
eine zweite Elektrode (432, 632), die in mindestens einem Teil einer Fläche des Gehäuses unterhalb des Anzeigefeldes gebildet ist, um der Außenseite der elektronischen Vorrichtung ausgesetzt zu sein;
ein transparentes Element (341, 420, 620), das zwischen dem Anzeigefeld (112, 240, 340) und der ersten Elektrode (431, 631) gebildet ist;
eine leitende Schicht (433, 633), die auf mindestens einem Teil einer Seitenfläche des transparenten Elements (341, 420, 620) gebildet ist, wobei ein Endstück der leitenden Schicht (433, 633) mit der ersten Elektrode (431, 631) elektrisch verbunden ist; und
einen Prozessor (1510), der konfiguriert ist, um über das Anzeigefeld (112, 240, 340) Führungsinformationen anzuzeigen, um einen Bereich des Anzeigefeldes anzuzeigen, der die erste Elektrode überlappt,
wobei die erste Elektrode (431, 631) auf mindestens einem Teil einer Fläche des transparenten Elements (341, 420, 620) gebildet ist, die der Außenseite zugewandt ist,
wobei mindestens ein Teil eines Grenzbereichs zwischen der Fläche und der Seitenfläche des transparenten Elements einen abgeschrägten Bereich bildet, und die erste Elektrode (431, 631) und die leitende Schicht (433, 633) sich zumindest teilweise in dem abgeschrägten Bereich überlappen, und
wobei, wenn die erste Elektrode (431, 631) in Kontakt mit einem ersten Körperteil eines Benutzers der elektronischen Vorrichtung ist, und die zweite Elektrode (432,632) in Kontakt mit einem zweiten Körperteil des Benutzers ist, die erste Elektrode und die zweite Elektrode elektrisch miteinander verbunden sind.

2. Elektronische Vorrichtung (100, 200, 300, 400, 600, 1500) nach Anspruch 1, welche ferner ein leitendes Element (533) umfasst, das mit einem verbleibenden Endstück der leitenden Schicht und einer Abtastschaltung elektrisch verbunden ist.

3. Elektronische Vorrichtung (100, 200, 300, 400, 600, 1500) nach Anspruch 1, wobei der Prozessor (1510) ferner konfiguriert ist, um mindestens einen Teil der Führungsinformationen durch einen verbleibenden Bereich des Anzeigefeldes anzuzeigen, welcher die erste Elektrode nicht überlappt.

4. Elektronische Vorrichtung (100, 200, 300, 400, 600, 1500) nach Anspruch 3, wobei der Prozessor ferner konfiguriert ist, um die Führungsinformationen unter Verwenden von grafischen Informationen anzuzeigen.

5. Elektronische Vorrichtung (100, 200, 300, 400, 600, 1500) nach Anspruch 1, welche ferner ein transparentes Element (341, 420, 620) umfasst, das mit dem Gehäuse verbunden ist, um das Anzeigefeld (112, 240, 340) abzudecken, wodurch eine Fläche der elektronischen Vorrichtung gebildet wird; und
eine Leiterplatte (321, 560), die in dem Gehäuse (111, 210, 310, 410, 610) untergebracht ist, und in der ein Regelkreis angeordnet ist, der mit der ersten Elektrode (431, 631) und der zweiten Elektrode (432, 632) elektrisch verbunden ist,
wobei die erste Elektrode (431, 631) im Wesentlichen transparent ist und auf einer Fläche des transparenten Elements (341, 420, 620) gebildet ist, die der Außenseite der elektronischen Vorrichtung zugewandt ist, und die zweite Elektrode (432, 632) auf einer Fläche des Gehäuses (111, 210, 310, 410, 610) gebildet ist, die der Fläche des transparenten Elements (341, 420, 620) gegenüberliegt; und
wobei der Regelkreis konfiguriert ist, um die biometrischen Informationen eines Benutzers unter Verwenden eines elektrischen Signals zu messen, das durch die erste Elektrode (431, 631) oder die zweite Elektrode (432, 632) erfasst wird.

6. Elektronische Vorrichtung (100, 200, 300, 400, 600, 1500) nach Anspruch 5, welche ferner ein leitendes Teil umfasst, das auf einer Seitenfläche des transparenten Elements (341, 420, 620) gebildet ist, um in Kontakt mit der ersten Elektrode (431, 631) zu sein.

7. Elektronische Vorrichtung (100, 200, 300, 400, 600, 1500) nach Anspruch 6, wobei mindestens ein Teil des leitenden Teils visuell undurchsichtig ist.

8. Elektronische Vorrichtung (100, 200, 300, 400, 600, 1500) nach Anspruch 6, welche ferner ein leitendes Verbindungselement umfasst, von dem ein Endstück mit dem leitenden Teil elektrisch verbunden ist, und von dem ein verbleibendes Endstück mit der Leiterplatte (321, 560) elektrisch verbunden ist.

9. Elektronische Vorrichtung (100, 200, 300, 400, 600, 1500) nach Anspruch 5, wobei der Regelkreis ferner konfiguriert ist, um eine Farbe der ersten Elektrode (431, 631) in Verbindung mit einem Messen der biometrischen Informationen zu verändern.

## Revendications

1. Dispositif électronique (100, 200, 300, 400, 600, 1500) comprenant :
un boîtier (111, 210, 310, 410, 610) ;
un panneau d'affichage (112, 240, 340) logé au moins partiellement dans le boîtier ;
une première électrode (431, 631) formée sur au moins une partie du panneau d'affichage de manière à être visuellement transparente et exposée à l'extérieur du dispositif électronique ;
une deuxième électrode (432, 632) formée dans au moins une partie d'une face du boîtier sous le panneau d'affichage de manière à être exposée à l'extérieur du dispositif électronique ;
un élément transparent (341, 420, 620) formé entre le panneau d'affichage (112, 240, 340) et la première électrode (431, 631) ;
une couche conductrice (433, 633) formée sur au moins une partie d'une face latérale de l'élément transparent (341, 420, 620), où une partie d'extrémité de la couche conductrice (433, 633) est connectée électriquement à la première électrode (431, 631) ; et
un processeur (1510) configuré pour afficher, via le panneau d'affichage (112, 240, 340), des informations de guidage pour indiquer une zone du panneau d'affichage qui chevauche la première électrode,
où la première électrode (431, 631) est formée sur au moins une partie d'une face de l'élément transparent (341, 420, 620), qui fait face à l'extérieur,
où au moins une partie d'une zone limite entre la face et la face latérale de l'élément transparent forme une zone chanfreinée, et la première électrode (431, 631) et la couche conductrice (433, 633) se chevauchent au moins partiellement dans la zone chanfreinée, et
où, lorsque la première électrode (431, 631) est en contact avec une première partie du corps d'un utilisateur du dispositif électronique et que la deuxième électrode (432, 632) est en contact avec une deuxième partie du corps de l'utilisateur, la première électrode et la deuxième électrode sont connectées électriquement l'une à l'autre.

2. Dispositif électronique (100, 200, 300, 400, 600, 1500) selon la revendication 1, comprenant en outre un élément conducteur (533) connecté électriquement à une partie d'extrémité restante de la couche conductrice et à un circuit de détection.

3. Dispositif électronique (100, 200, 300, 400, 600, 1500) selon la revendication 1, où le processeur (1510) est en outre configuré pour afficher au moins une partie des informations de guidage à travers une zone restante du panneau d'affichage, qui ne chevauche pas la première électrode.

4. Dispositif électronique (100, 200, 300, 400, 600, 1500) selon la revendication 3, où le processeur est en outre configuré pour afficher les informations de guidage en utilisant des informations graphiques.

5. Dispositif électronique (100, 200, 300, 400, 600, 1500) selon la revendication 1 comprenant en outre un élément transparent (341, 420, 620) couplé au boîtier pour recouvrir le panneau d'affichage (112, 240, 340), formant ainsi une face du dispositif électronique ; et
une carte de circuit imprimé (321, 560) logée dans le boîtier (111, 210, 310, 410, 610), et ayant un circuit de commande disposé à l'intérieur connecté électriquement à la première électrode (431, 631) et à la deuxième électrode (432, 632),
où la première électrode (431, 631) est substantiellement transparente et est formée sur une face de l'élément transparent (341, 420, 620), qui fait face à l'extérieur du dispositif électronique et la deuxième électrode (432, 632) est formée sur une face du boîtier (111, 210, 310, 410, 610), qui est opposée à la face de l'élément transparent (341, 420, 620) ; et
où le circuit de commande est configuré pour mesurer les informations biométriques d'un utilisateur en utilisant un signal électrique acquis via la première électrode (431, 631) ou la deuxième électrode (432, 632).

6. Dispositif électronique (100, 200, 300, 400, 600, 1500) selon la revendication 5, comprenant en outre une partie conductrice formée sur une face latérale de l'élément transparent (341, 420, 620) pour être en contact avec la première électrode (431, 631).

7. Dispositif électronique (100, 200, 300, 400, 600, 1500) selon la revendication 6, où au moins une partie de la partie conductrice est visuellement opaque.

8. Dispositif électronique (100, 200, 300, 400, 600, 1500) selon la revendication 6, comprenant en outre un élément de connexion conducteur, dont une partie d'extrémité est connectée électriquement à la partie conductrice et dont une partie d'extrémité restante est connectée électriquement à la carte de circuit imprimé (321, 560).

9. Dispositif électronique (100, 200, 300, 400, 600, 1500) selon la revendication 5, où le circuit de commande est en outre configuré pour changer une couleur de la première électrode (431, 631) en relation avec une mesure des informations biométriques.
